# EUROPEAN PATENT APPLICATION

(11) **EP 2 478 849 A2**
(43) Date of publication of application: **25.07.2012**
(21) Application number: 12156139.3
(22) Date of filing: 18.03.2009
(51) Int. Cl.: A61B 17/115

(54) **Endoscopic stapling device**

(30) Priority: 18.03.2008 US 50169
(62) Divisional of application: 09722297.0
(71) Applicant: Barosense, Inc., Redwood City, CA 94063 (US)
(72) Inventor: Cole, David, San Mateo, CA California 94403 (US); Smith, Andrew, Ann Arbor, MI Michigan 48103 (US)
(74) Representative: Millburn, Julie Elizabeth

(57) **Abstract**

Described herein is a stapling device and method used to apply one or more fasteners to body tissue. In one embodiment, a fastener-applying device, which is preferably a stapler, is passed transorally into the stomach and used to plicate stomach tissue by engaging tissue from inside of the stomach and drawing it inwardly. In the disclosed embodiments, the tissue is drawn inwardly into a vacuum chamber, causing sections of serosal tissue on the exterior of the stomach to be positioned facing one another. The disclosed staplers allow the opposed sections of tissue to be moved into contact with one another, and preferably deliver staples for maintaining contact between the tissue sections at least until serosal bonds form between them. Each of these steps may be performed wholly from the inside of the stomach and thus can eliminate the need for any surgical or laparoscopic intervention. After one or more plications are formed, medical devices may optionally be coupled to the plication(s) for retention within the stomach.

## Description

### FIELD OF THE invention

The present invention relates generally to the field of systems and methods for performing endoscopic surgery, and specifically to systems and methods for endoscopic stapling of tissue within body cavities,

### BACKGROUND OF THE INVENTION

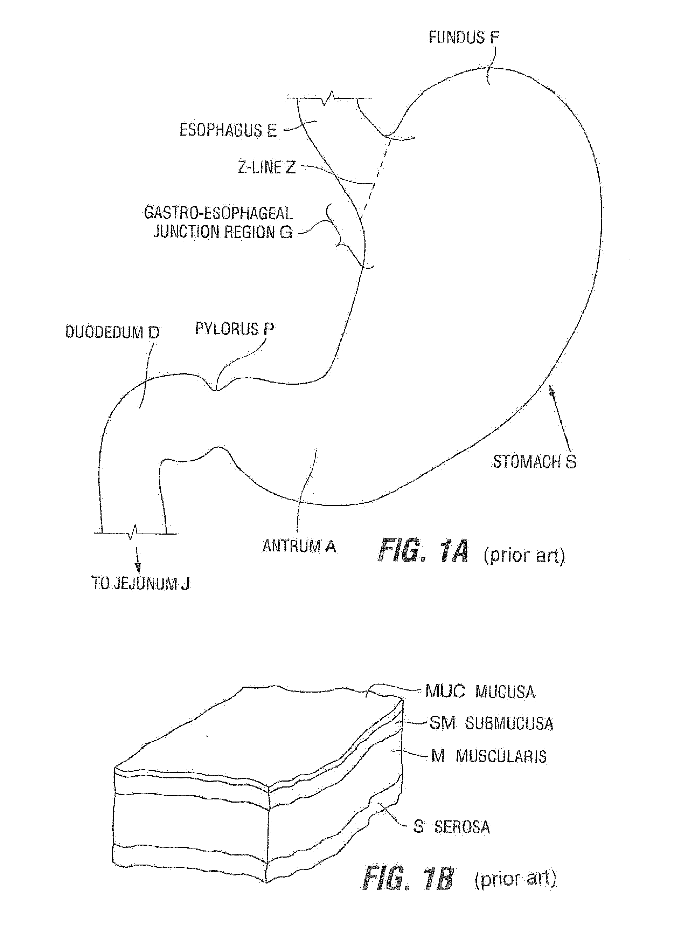

An anatomical view of a human stomach S and associated features is shown in Fig. 1A. The esophagus E delivers food from the mouth to the proximal portion of the stomach S. The z-iine or gastro-esophageal junction Z is the irregularly-shaped border between the thin tissue of the esophagus and the thicker tissue of the stomach wall, The gastro-esophageal junction region G is the region encompassing the distal portion of the esophagus E, the z-fine, and the proximal portion of the stomach S.

Stomach S includes a fundus F at its proximal end and an antrum A at its distal end. Antrum A feeds into the pylorus P which attached to the duodenum D, the proximal region of the small intestine. Within the pylorus P is a sphincter that prevents backflow of food from the duodenum D into the stomach. The middle region of the small intestine, positioned distally of the duodenum D, is the jejunum J.

Fig. 1B illustrates the tissue layers forming the stomach wall. The outermost layer is the serosal layer or "serosa" S and the innermost tayer, lining the stomach interior, is the mucosal layer or "mucosa" MUC. The submucosa SUM and the mufti-layer muscularis M lie between the mucosa and the serosa.

There are a number of applications for endoscopic application of fasteners such as staples to tissue within a body cavity. Some of those applications involve forming tissue structures such as plications or folds in tissue of the body cavity.

Several prior applications, including International Application No. WO 2005/037152 having an international filing date of October 8, 2004 and U.S. Application No. 11/439,461, filed May 23, 2006 (both incorporated herein by reference) describe methods according to which medical implants are coupled to tissue structures formed within the stomach. According to these application, devices for inducing weight loss (e.g. by restricting and/or obstructing flow of food into the stomach, and/or by occupying a portion of the stomach volume) may be coupled to tissue tunnels or plications Formed from stomach tissue.

For example, U.S. Application No. 11/439,461 describes a restrictive and/or obstructive implant systems for inducing weight toss. In one embodiment, flexible loops are coupled to tissue plications formed in the gastroesophageal junction region of the stomach. An implant, such as a flow restrictive and/or obstructive implant, is passed through the loops 2 and thus retained in the stomach.

In other instances, tissue plications may themselves be sufficient to provide the necessary treatment. For example, the plications may be used to reduce stomach volume or form a flow restriction within the stomach as disclosed in WO 2005/037152 and in Applicants' co-pending Application No. 11/542,457, filed October 3, 2006, U.S. Publication No. 2007-0219571, which is incorporated herein by reference.

Other types of implants may be coupled to such plications or other tissue structures for a variety of purposes. These implants include, but are not limited to prosthetic valves for the treatment of gastro-esophageal reflux disease, gastric stimulators, pH monitors and drug eluting devices that release drugs, biologics or cells into the stomach of elsewhere in the GI tract. Such drug eluting devices might include those which release leptin (a hormone which creates feelings or satiety). Ghrelin (a hormone which creates feelings of hunger), octreolide (which reduces Ghrelin levels and thus reduces hunger). Insulin, chemotherapeutic agents, natural biologics (e.g. growth factor, cytokines) which aid in post surgery trauma, ulcers, lacerations etc. Still other implants might be of a type which might provide a platform to which specific cell types can adhere, grow and provide biologically-active gene products to the GI tract, and/or a platform for radiation sources that can provide a local source of radiation for therapeutic purposes, or provide a platform whereby diagnostic ligands are immobilized and used to sample the GI tract for evidence of specific normal or pathological conditions, or provide an anchor point for imaging the GI tract via cameras and other image collecting devices.

The prior application listed above, address the desirability of forming tissue plications, pockets or tunnels in a way that regions of serosal tissue (i.e. the tissue on the exterior surface of the stomach) are retained in contact with one another. Over time, adhesions formed between the opposed serosal layers create strong bonds that can facilitate retention of the plication/pocket/tissue over extended durations, despite the forces imparted on them by stomach movement and implanted devices.

Regardless of the application for which a plication is being formed, it is highly desirable to from that plication using steps carried out from within the stomach using instruments passed down the esophagus, rather than using more invasive surgical or laparoscopic methods. The present application describes endoscopic staplers which may be passed transorally into the stomach and used to form serosal-to-serosal plications in a stomach wall,

### SUMMARY OF THE INVENTION

In one aspect, the invention includes a stapler device (12) for applying a staple to a tissue. The device include (i) a first or staple member (25) having a first-member or staple housing (28) and a staple holder (78) that is independently movable with respect to the staple housing, (ii) a second or anvil member (27) having as second-member or anvil housing (30) and an anvil (96) carried on the anvil housing (30), and (iii) a drive assembly (29) including a drive member (68) operatively connected to the staple holder (78) for movement within the staple housing (28) from a first, retracted position to a second, extended position, and an arm assembly (32) operatively Coupled to the staple and anvil members (25, 27), such that movement of the drive member (68) from its retracted to its extended position is effective to (i) move the staple holder (78) with respect to the staple housing (28) toward the anvil (96) (Al), and (ii) move the anvil member (27) toward the staple member (A2),

The anvil (96) may be independently movable within the anvil housing (30), and the arm assembly (32) may be operatively coupled to the anvil (96), such that movement of the drive member (68) from its retracted to its extended second position is effective to (i) move the staple holder with respect to the staple housing toward the anvil (A1), (ii) move the anvil member toward the staple member (A2), and (iii) move the anvil with respect to the anvil housing toward the staple holder (A3).

The anvil member (27) may include drive links (114) operatively connected to the arm assembly (32) for moving the anvil (96) toward the staple holder (78) (A3) as that drive assembly (106) is moved from its retracted to its extended position.

Alternatively, the anvil may be independently driven with the anvil housing toward the staple holder by an independent hydraulic drive within the anvil housing. The anvil drive motion preferably occurs as the staple holder is being driven within its housing.

The staple holder (78) and anvil (96) may have confronting surfaces that define, in combination with arm assembly (32), a chamber (21), and the effect of movement of the drive member (68) from its first to second positions is to squeeze tissue captured within the chamber to form a compressed tissue fold.

The chamber may be covered by a membrane, such as an elastomeric or pleated membrane (24), allowing tissue to be drawn into the chamber, with application of a vacuum to the membrane. The elastomeric member may have an opening an opening (26) on one side thereof, for sucking tissue into the chamber, and movement of the drive member (68) from its retracted to extended position, with application of vacuum to the chamber, its effective to draw tissue into the side of the chamber opposite the opening.

The drive member may includes a disc (68) that travels within the staple housing (25), and carries at least one pin (84) that moves within a slot (64) in the staple housing (25), limiting the extent of travel of the drive member toward its extended position to the extent of travel allowed for the pin (84) within the slot (64). The device may further include at least one arm spreader (113) pivotally connecting disc (68) to the arm assembly (32), for spreading the arm assembly outwardly as the disc travels from its retracted to its extended position.

The drive member may include a drive piston (106), and may further includes a staple piston (116) carried for movement within the drive piston (106) between a first, retracted and a second, extended positions and a staple pusher (76) attached to drive piston for engaging one or more staples in the staple holder (78) and eject the one or more staplers from the holder against the anvil (96), when the staple pusher is moved with the staple piston (116) from a retracted to an extended position.

The staple bolder (78) may be designed to hold an annular array of staples, and the staple pusher (78) may be designed to engage and eject the array of staples in the holder simultaneously. The staple holder may be a replaceable staple cartridge adapted to be inserted into the staple housing (28) to travel therein with the drive member (68) between retracted and extended positions. The drive member may include a disc (68) having at least one axially extending pest (84) adapted to engage the staple cartridge, with such received in the device, and prevent angular movement cat' the cartridge within the first-member housing.

The device may further include a cartridge-,side reinforcing ring (83) disposed against the cartridge (78), and having openings (85) for receiving staples therethrough, for attaching the reinforcing ring (83) to the side of stapled tissue facing the cartridge.

The device may further include a tissue cutter (86) mounted on the staple piston (116), adapted to cut a hote in a tissue fold held between the staple holder (78) and anvil (96), as the tissue fold is being stapled by movement, of the staple piston (116) from its retracted to its extended position.

The second member (30) may include a compressible cutting board (99), allowing the cutter (86) to be advanced by movement, of the staple piston (116) beyond its point of initial contact with the cutting board. The cutting board (99a) may be formed of a material, such as silicone, that can be penetrated by the cutter. In another embodiment, the cutting board is (99d) is spring biased in the direction opposing movement of the cutter.

The device may further include an alignment pin (160, 168) carried on one of the staple and anvil members, and a pin-receiving bushing (164, 170) carried on the other of the members, where the pin and bushing are positioned such that movement of the staple piston toward its extended position causes the pin to mate with the bushing, thus to hold the two members in axial alignment as staples are ejected with further movement of the staple piston 1 16) toward its extended position. One of the pin or pin-receiving bushing may be may be retractable under a spring bias.

The device may be carried at the distal end of a shaft (16) carrying hydraulic fluid to the device, and the staple, and anvil members (25, 27) of the device may be proximal and distal members, respectively, with the shaft being operatively connected to the proximal member of the device.

Also disclosed is a method for capturing and stapling a tissue fold, by the steps of:
(a) drawing a tissue fold into a vacuum chamber 21 defined by first and second relatively movable members (25, 27) and a membrane (24) extending between the two,
(b) advancing a staple holder (78) and anvil (96) contained within the first and second, members (25, 27), respectively, toward one another by independent movement of the staple holder and anvil within its associated member, respectively, and by moving the second housing (27) toward the first housing (25);
(c) by continued application of vacuum during said advancing, continuing to draw tissue into said chamber (21) unfit a tissue fold is captured between the staple holder and anvil, and
(d) stapling the captured tissue fold.
In a related aspect, the invention includes medical instrument (10) for stapling stomach tissue. The instrument is comprised of the stapling device (14) described above, a shaft (16) having a proximal-end handle and a distal end, an articulating section (128) connecting the distal end of the shaft to the first, proximal member of the stapling device, and a hydraulic fluid line (130) contained within the shaft capable of carrying hydraulic fluid under a pressure of up to 1,500 psi or greater, to the drive mechanism (106) in the staple member (25), wherein the hydraulic fluid line within the articulating section has a coiled or sinusoidal configuration, to accommodate off-axis movement of the device with respect to the shaft.

The articulating section (128) may have a spine (128) formed of a plurality of links (132) formed over the coiled or sinusoidal portion of the hydraulic line (130). The stapling device may include a hydraulically driven drive member (68) and a hydraulically driven staple piston (116), and the shaft (16) and articulating section (128) may carry separate hydraulic lines (130) for the drive member and staple piston, and the lines (130) may have an interleaved coil configuration within the articulating section.

In a related aspect, the invention provides a snap-on connection between the distil end of a shaft and the proximal face of a staple member in a hydraulically activated staple device, for quick release or operative attachment of the shaft to the staple device. The connection may include, at the distal shaft end, a plate having one or more openings for supplying hydraulic fluid under pressure from hydraulic fines attached to the openings at the input side of the plate, and where the plate includes an engagement edge adjacent each opening. The plate-recerving face of the staple device includes correspondingly positioned openings for receiving fluid into the device, where the openings communicate at the output side of the face with hydraulic supply lines within the staple device, and where the proximal face includes an undercut boss adjacent each opening. In operation, the shaft plate is placed against the staple-device face, and rotated slightly to wedge the engagement, edge(s) on the shaft plate within the undercut regions of the bosses of the device plate, compressing an O-ring located between the plate and face at each opening, thus sealing the connection between the aligned openings. A lock mechanism on the shaft plate engages the device face to lock the plate against rotational movement once the fluid-supply opening are received.

In another aspect, the invention includes stapler device (14) for applying a Staple to a tissue. The device comprise a. first, staple member (25) having a staple housing (28) and a staple holder (78), a second, anvil member (27) having an anvil housing (30) and an anvil (96), and a drive assembly including (i) a drive member (68) for movement within the staple housing (28) from a retraced position to an extended position, (ii) attached to the drive member, one or more pins (84) that travel within one or more slots (64) formed in the staple housing, limiting movement of the drive member between retracted and extended positions to the limits oaf travel of said pin(s) in said slot(s), (iii) an arm assembly (32) operatively coupled to the staple and anvil members, such that movement of the drive member (68) from its retracted toward its extended position is effective to move the staple holder toward the anvil (A1), and (iv) at least one arm spreader (113) pivotally connecting said drive member to the arm assembly, for spreading the ann assembly outwardly as the drive member travels from its first to its second position.

The arm spreader (113) may be pivotally connected to the pin and the arm assembly, such that movement of the pin within its slot in a retracted to extended position is effective to move the arm assembly outwardly. The arm spreader may thus provide a truss-like support between the staple body and the associated assembly arm

The drive member (68) may be effective to advance the staple holder (78) within its housing (28), as the drive member is moved from its retracted to extended positions, and the arm assembly (32) may be effective to advance the anvil (96) within its housing as the drive member is moved from its retracted to extended positions.

In still another aspect, the invention includes a stapler device (14) for forming a cut within a tissue. The device includes a first member 25 having a housing 28 and driven piston (116) and a cutter (86) attached to the piston for movement therewith between retracted and extended positions, and a second member having a housing (30) and a cutting board (99). The first, member has a drive piston (116) for moving the cutter (86) from a retracted to an extended position, where the cutter (86) contacts the cutting board (99) to form a hole in a tissue disposed between the two members, and the cutting board (99) has a resilient cutting .surface that allows the cutter (86) to advance in the direction of the cutting board (99) beyond the initial point of contact therewith. The cutting board (99a) may be formed of a material, such as silicone, that can be penetrated by the cutter. In another embodiment, the cutting board (99d) is spring biased in the direction opposing movement of the cutter toward its extended position.

In another aspect, the invention includes a stapler device (14) for applying a staple to a tissue. The device comprises a first, staple member having a first or staple housing (28) and a staple holder (78), a second, anvil member having a second, anvil housing (30) and an anvil (96), a drive assembly including (i) a drive member (68) for movement within the staple member housing from a retracted position to an extended position, to move the staple holder adjacent the anvil for capturing tissue between the holder and anvil, a staple piston (116) in the first member, movable between first, retracted and second, extended positions in the first member for driving staples from the holder through the tissue against the anvil, an alignment pin (160, 168) carried on one of the staple and anvil members, and a pin-receiving bushing (164, 170) carried on the other of the members, where the pin and bushing are positioned such that movement of the staple piston toward its extended position causes the pin to mate with the bushing, thus to hold the two members in axial alignment as staples are ejected with further movement of the staple piston (116) toward its extended position.

One of the pin (160, 168) and pin-receiving bushing (164,170) may be retractable under a spring bias.

The drive member (68) may be effective to advance the staple holder (78) within its housing (28), as the drive member is moved from its retracted to extended positions, and the arm assembly (32) may be effective to advance the anvil) (96) within its housing as the drive member is moved from its retracted to extended positions.

In still another aspect, the invention includes a tissue capture device (14) for capturing and immobilizing a tissue fold. The device incudes a first member (25) having a housing (30) and a first tissue-contact plate (78) independently movable within the first-member housing, and a second member (27) having a housing (30) and a second tissue-contact plate (96) independently movable within the second-member housing. A driven assemb!y (29) in the device, includes a drive member (68) operatively connected to the first tissue-contact plate (78) for movement within the first-member housing (28) from a retracted position to an extended position, and an arm assembly (32) operatively coupled to the first and second members (25, 27), such that movement of the drive member (68) from its retracted to its extended position is effective to (i) move the tissue-contact plate (78) within the first-meniber housing toward the second-tissue contact member (96) (A1) and (ii) move the anvil member (27) toward the staple member (A2). A membrane, such as an elastomeric or pleated membrane, extends between the two members, defining a tissue capture chamber between the two tissue-contact plates, and having an opening therein for drawing tissue into the chamber, upon application of a vacuum to the chamber. An expansion member or raiser (37) operatively coupled to the two members operates to expand for the chamber membrane outwardly, on the side of the membrane opposite the opening, upon movement of the driven member from its retracted to its expanded condition, causing additional tissue to be drawn into the chamber between the two tissue-contact plates, until the tissue is captured by clamping between two tissue-contact plates.

Also disclosed is a method for capturing a tissue fold by the steps of:
(a) drawing tissue (17) into a vacuum chamber (21) defined by first and second relatively movable members (25, 27) and a membrane, such as an elastomeric or pleased membrane 24), extending between the two,
(b) advancing a first tissue-contact plate (78) and a second tissue-contact place (96) contained within the first and second members, respectively, toward one another by independent movement of the First and second tissue-contact plates (78, 96) staple within their associated members (25, 27, respectively, and by moving the second member toward the First member;
(c) by continued application of vacuum during said advancing, continuing to draw tissue into said chamber until a tissue fold (17a) is captured between the first and second tissue-contact plates.

These and other objects and features of the invention will become more fully apparent when the following detailed description of the invention is read in conjunction with the accompanying drawings.

### BRIEF DESCRIPTION OF THE DRAWINGS

Fig. 1A is a schematic illustration of a human stomach and a portion of the small intestine, as known in the prior art.

Fig. 1B is a cross-sectioiial perspective view of a portion of a stomach wall, illustrating the layers of tissue Forming the wall, also as known in the prior art.

Fig. 2 illustrates an endoscopic stapling system of instrument constructed in accordance with an embodiment of the invention.

Figs. 3A - 3C are perspective views showing the stapler head or device of the stapling system of Fig. 2 in three different positions.

Fig. 4 is a perspective view of the stapler head or device, with the membrane removed, showing first and second members in the device.

Fig. 5 is a perspective view of the proximal end of the staple housing of the stapler device of Fig. 4.

Fig. 6 is a perspective view of the distal end of the staple housing of the stapler device of Fig. 4.

Fig. 7 is an exploded perspective view showing elements advanceable within the staple housing during compression and stapling operation.

Fig. 8 is plan view of a staple reinforcement device.

Fig. 9 is a side elevation view of a staple cartridge.

Fig. 10 is a perspective view of the staple housing similar to Fig. 6, but showing some of the elements of Fig. 7 within the housing.

Figs. 11A - 11D are a series of schematic representation of the hydraulic chamber and pistons, illustrating operation of an exemplary hydraulic system during tissue compression and stapling.

Fig. 11E is similar to Fig. 11D and shows an alternative piston configuration.

Fig. 12 is a perspective view of the anvil housing of the stapler head of Fig. 4.

Fig. 13 is a perspective view of the anvil support.

Fig. 14 is a plan view of the anvil.

Fig. 15A is a cross-sectional side view of the cutting device and a first embodiment of a cutting board.

Fig. 15B is a cross-sectional side view of the cutting device and a second embodiment, of a cutting hoard.

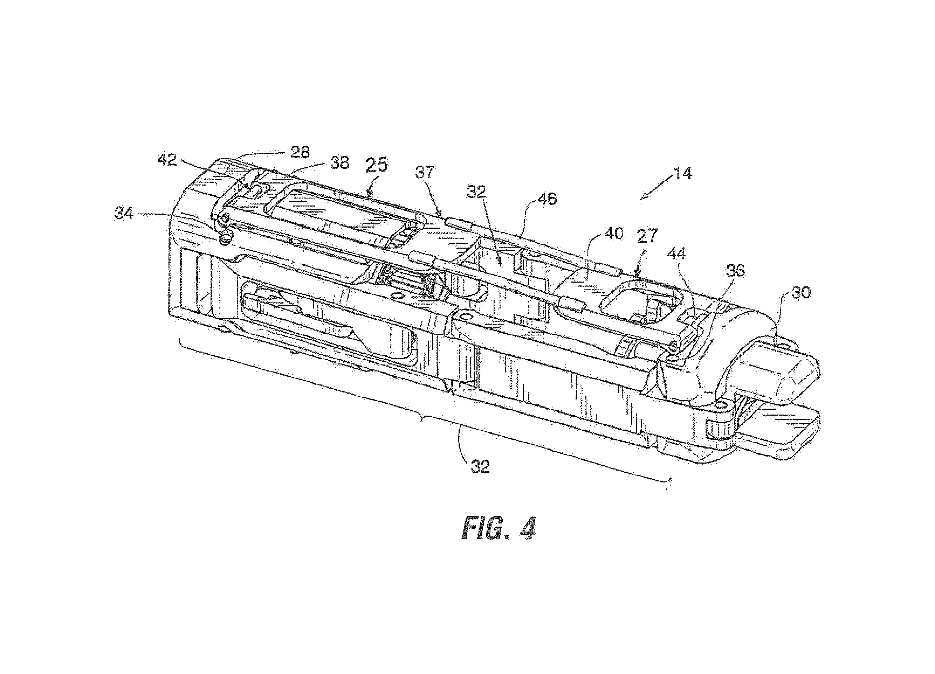

Fig, 16 is a perspective view of the hinged arm assemblies of the stapler head of Fig. 4.

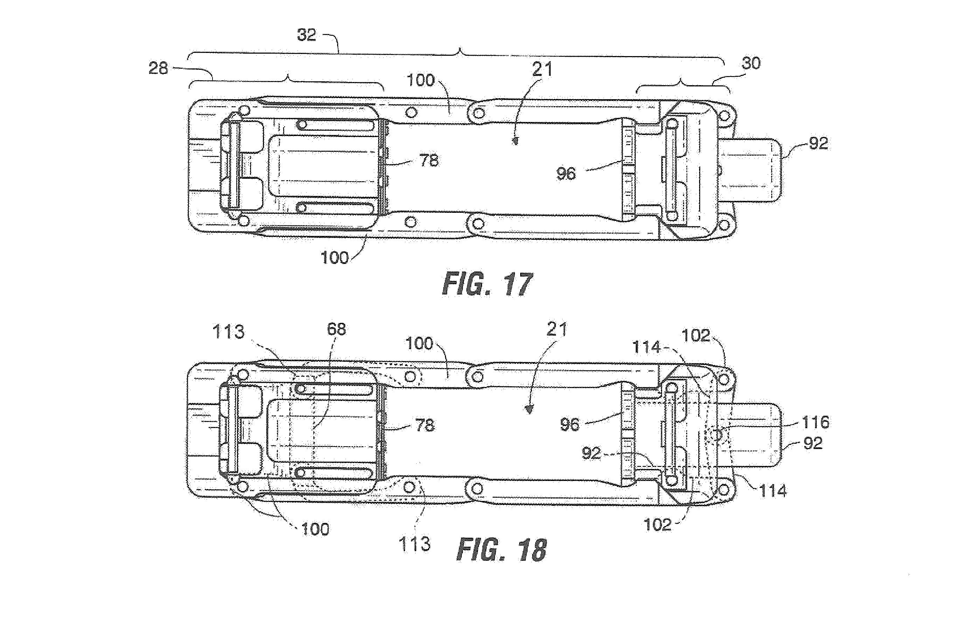

Fig. 17 is a top plan view of the stapler head of Fig. 4 in the streamlined position for introduction into the body. Both the membrane and the membrane raiser are not shown for purposes of clarity.

Fig. 18 is similar to Fig. 17 and illustrates hidden features of Fig, 17.

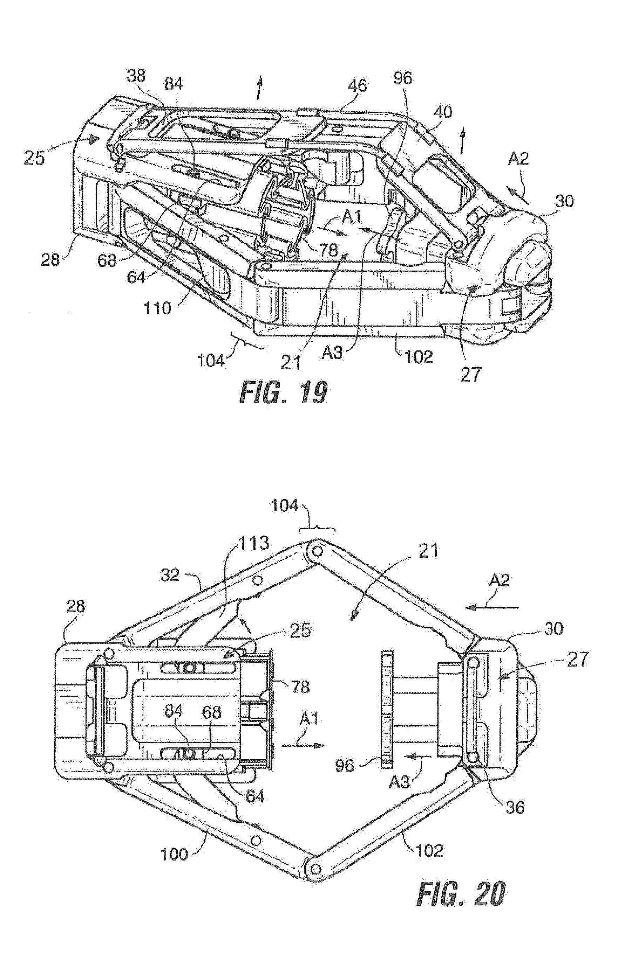

Fig. 19 is a perspective view of the stapler head in an intermediate, partially expanded, position.

Fig. 20 is a plan view simitar to Fig. 17 but showing the stapler head in the intermediate position.

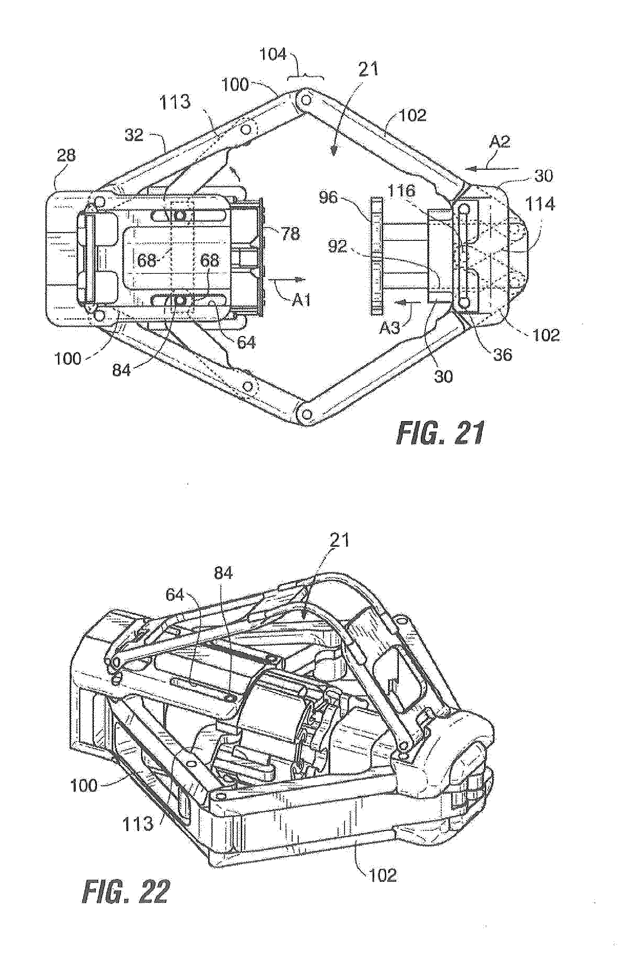

Fig. 21 is similar to Fig. 20 and illustrates hidden features of Fig. 20.

Fig. 22 is a perspective view of the stapler head in a fully expanded, full compression position.

Fig, 23 is a plan view simitar to Fig. 20 but showing the stapler head in the full compression position.

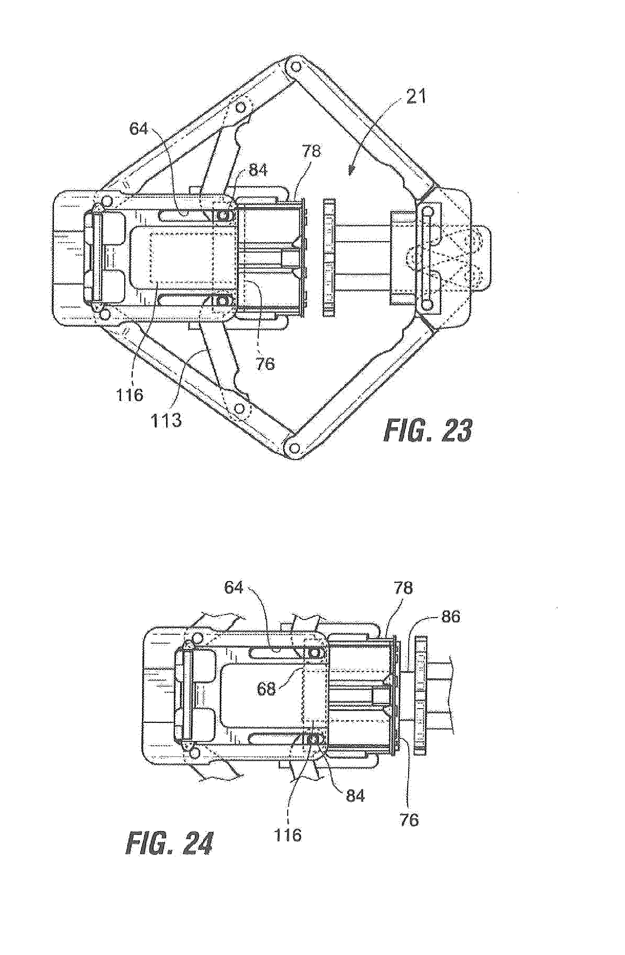

Fig. 24 is similar to Fig. 23 and illustrates hidden features of Fig. 24.

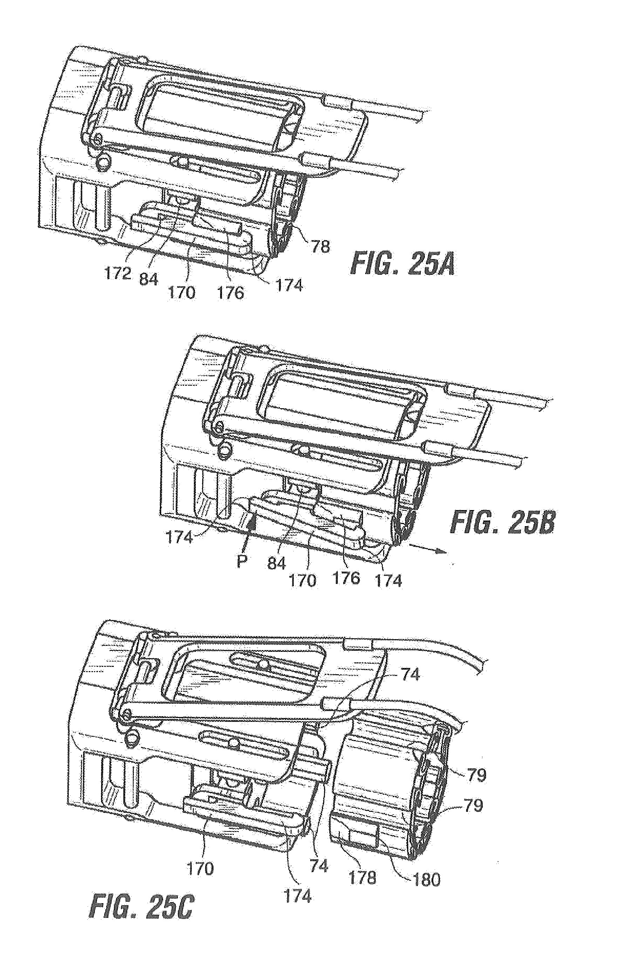

Figs. 25A - 25B are perspective views showing the staple housing, cartridge and a portion of the membrane raiser. These figures illustrate the steps of detaching a staple cartridge from the staple housing.

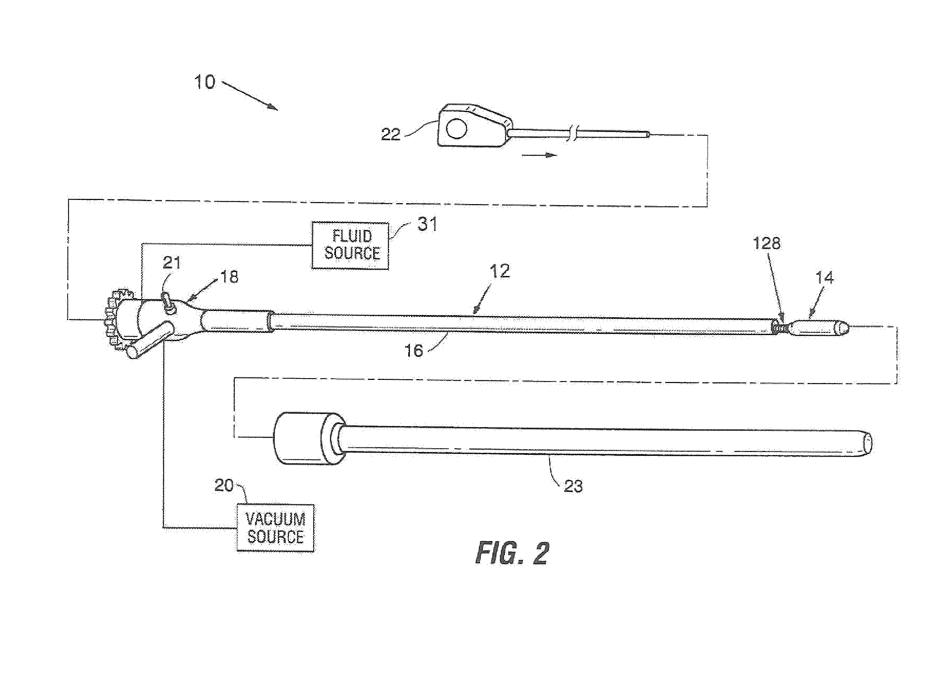

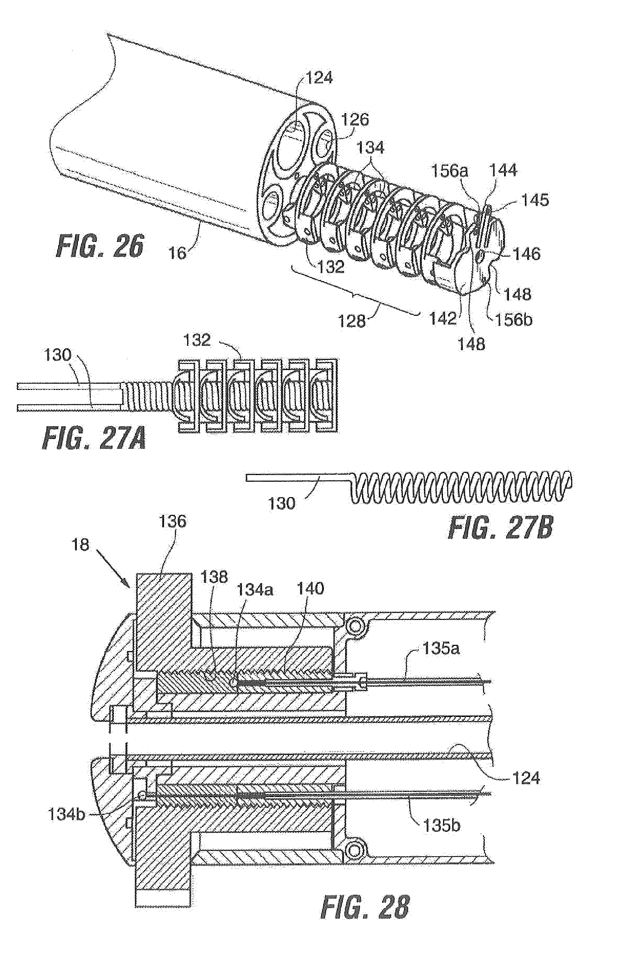

Fig. 26 is a perspective view of the stapler instrument of Fig. 2, with the staple head removed.

Fig. 27A is a plan view of the articulating section of the stapler of Fig. 2. showing interleaved drive fluid lines.

Fig. 27B shows a drive fluid line having an alternate longitudinally expandable shape.

Fig. 28 is a cross-sectional side view of the handle of the stapler instrument of Fig. 2.

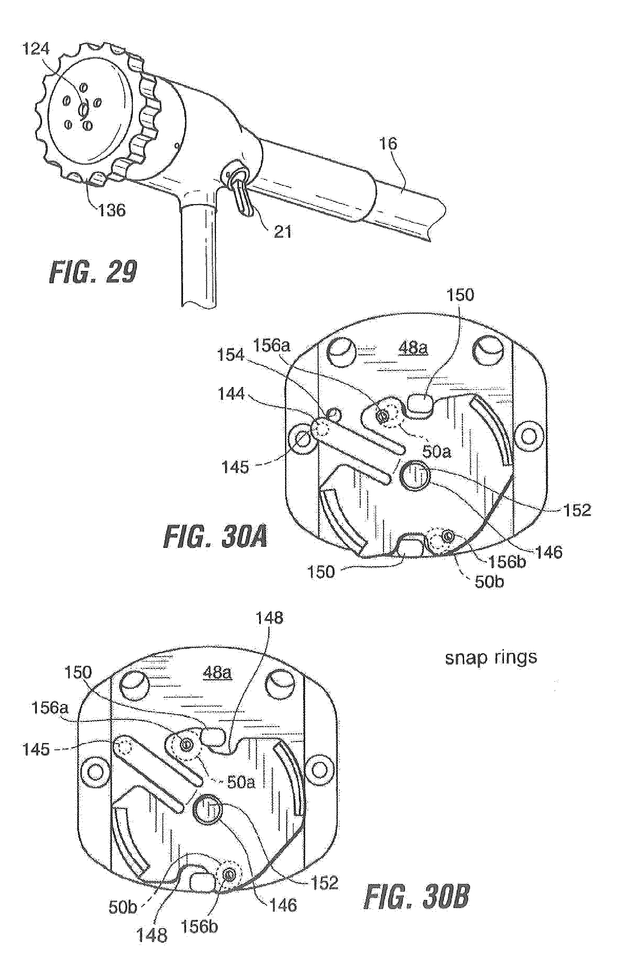

Fig. 29 is a perspective view of the handle of the stapler of Fig. 2.

Figs. 30A and 30B are plan views of the proximal face of the staple housing, showing a method for attaching the end plate of the stapler handle to the staple housing.

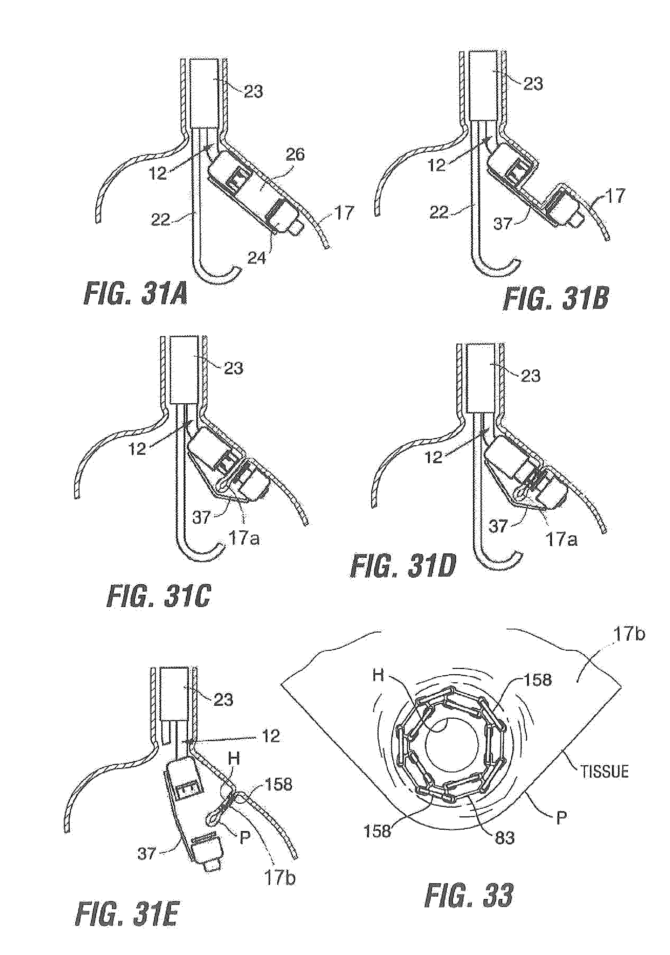

Figs. 31A - 31 E are a series of drawings schematically illustrating use of the system of Fig. 2 to form a plication in a stomach.

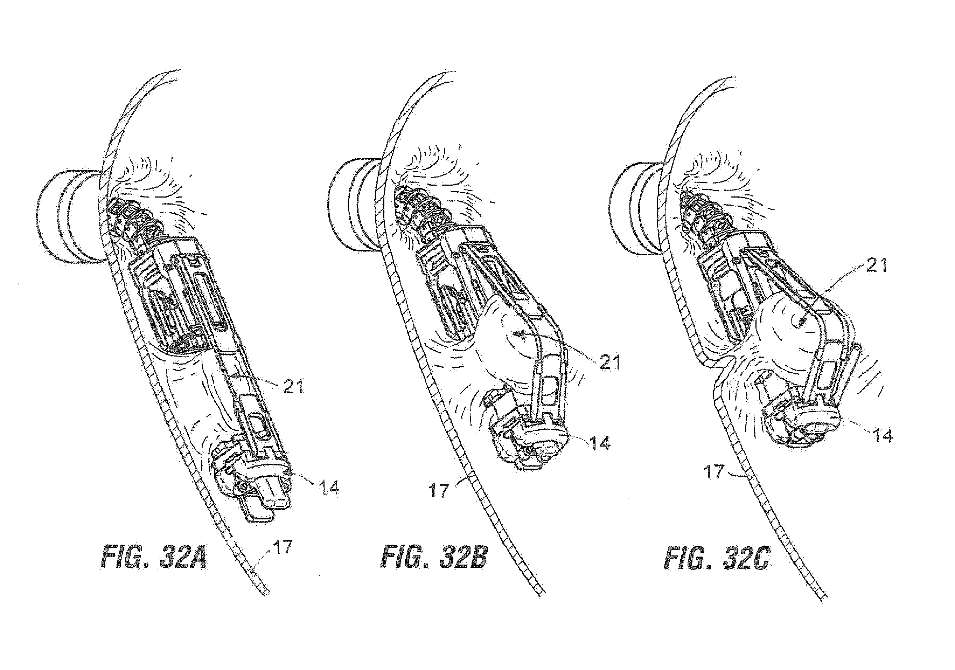

Figs. 32A-32C are a series of perspective views illustrating use of the stapler of Fig. 2 to acquire, compress, and then staple stomach wall tissue to form a plication in the stomach. The membrane is not shown in these drawings.

Fig. 33 is a top pian view of a plication formed in body tissue.

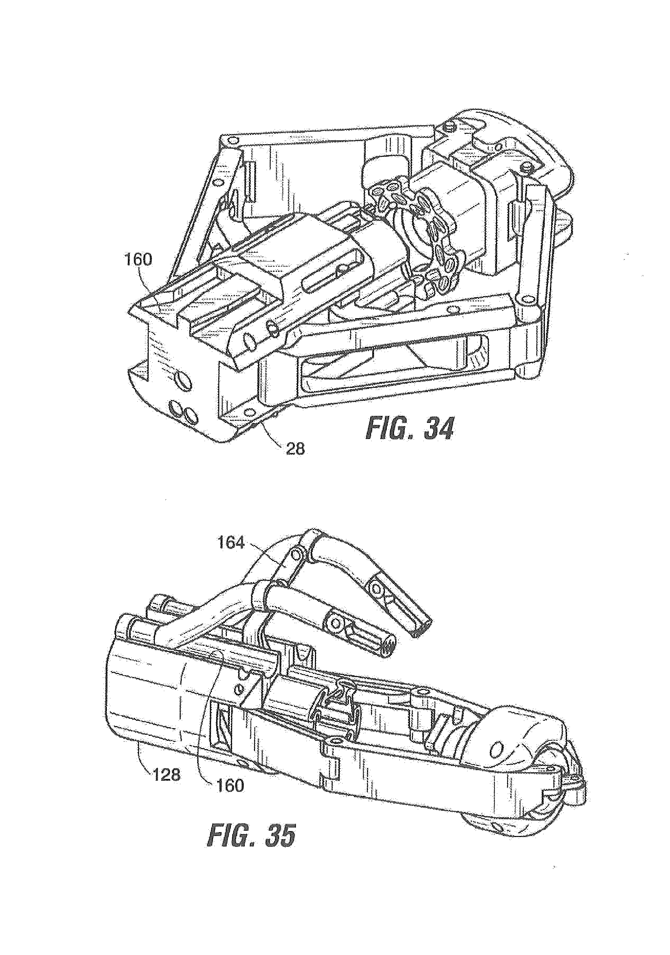

Figs. 34 and 35 are perspective views of an alternative stapler head equipped to carry additional toots.

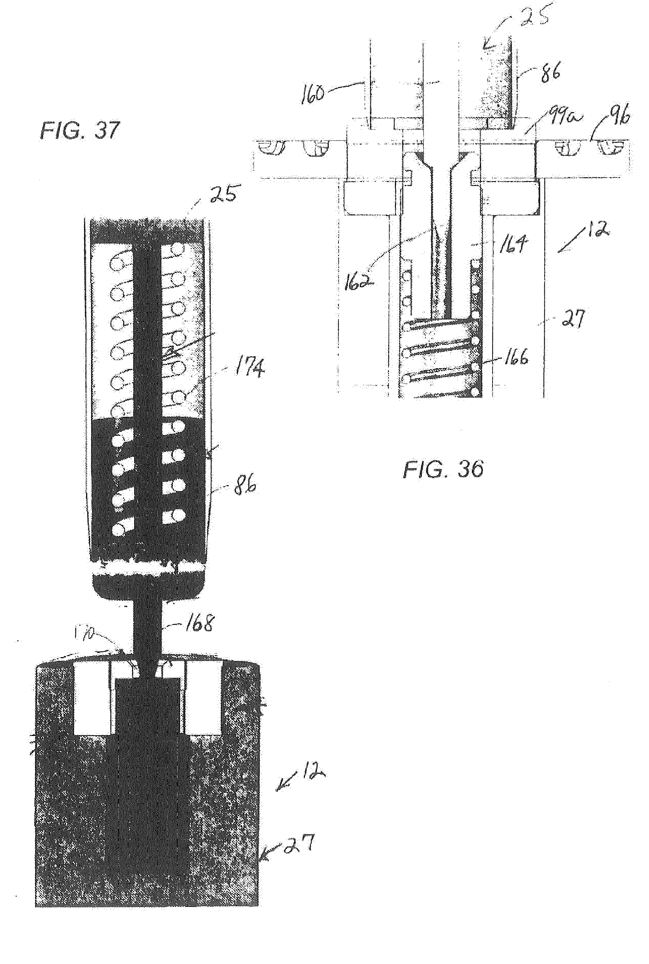

Figs. 36 and 37 show alternative embodiments of stapfer-aiignment structure in the stapler device of the invention.

### DETAILED DESCRIPTION OF THE DRAWING

The present application describes endoscopic fastener-applying devices which in preferred embodiments may bye passed transorally into the stomach and used to plicate stomach tissue.

In the disclosed embodiments, tissue is drawn inwardly into a vacuum chamber, although tissue may be drawn inwardly using other components (e.g. gaspers) that do not involve the use of a vacuum. When a portion the interior stomach watt is drawn inwardly, sections of serosal tissue on the exterior of the stomach are positioned facing one another. The disclose fastener applying device allows the opposed sections of tissue to be moved into contact with one another, and delivers fasteners that will hold the tissue sections together until at least such time as serosal bonds form between them. Each of these steps may be performed wholly from the inside of the stomach and thus can eliminate the need for any surgical or laparoscopic intervention. After one or more plications is formed, medical devices (including, but not limited to any of the types listed above) may be coupled to the plication(s) for retention within the stomach.

The disclosed embodiments include an optional feature that form a hole or cut in a plication using the fastener-applying device. This hole or cut might be formed so that a portion of a medical implant may be passed through or linked to the hole/cut, or it may be formed so as to provoke a healing response that will contribute to the strength of the resuming tissue bond.

In the description of the embodiments given below, the fastener-applying devices are described as being staplers, and exemplary method are given with respect to the formation of plications in stomach tissue. It should be understood, however, that the embodiments described herein include features having equal applicability for applying other types of fasteners, and for applying staples or other fastener for purposes other than formation of plications. More specifically, the term "staple" is used herein to designate any type of fastener that (i) can be pushed through tissue, and (ii) has one or more leg members that when forced, against an anvil are crimped to secure the fastener to the tissue and hold tissue fastened tissue fold together. The disclosed embodiments and methods will also find use in parts of the body outside the GI systems. Additionally, although the disclosed embodiment features circular stapling and cutting of a concentric hole, modifications are conceivable in which linear stapling can be accomplished, as well as circular or linear stapling without cutting.

Fig. 2 illustrates one embodiment of a system or instrument 10 for tissue stapling that is suitable for endoscopic use, as well as surgical or laparoscopic use if desired.

Generally speaking, system 12 includes a stapler or stapler instrument 12 having a stapler head or device 14 positioned on a distal portion of a shaft 16, A handle 18 on the shaft 16 controls articulation of the stapler hend 14 and actuation of the tissue acquisition, tissue compression, and stapling function of the stapler head 14. Vacuum and fluid sources 20, 31 in the system are fluidly coupled to the handle 18 for use in tissue acquisition, compression and stapling as discussed below, The vacuum source 20 may be the "house vacuum" accessible through a coupling on the wall of the operating room, or an auxiliary suction pump. The stapler may include a switch 21 allowing the user to control airflow between the vacuum source and stapler.

The stapler device also serves to capture a tissue fold for stapling, and is thus also referred to herein as a tissue capture device for immobilizing a tissue fold, e.g., for fastening the sides of the fold. The tissue capture device may operate independently for capturing tissue, e.g., absent a separate stapling mechanism, or may be combined with the stapling elements, as illustrated.

The fluid source 31 may be a single source of drive fluid (e.g. water, saline, oil, gas) or multiple sources, but in each case the fluid source preferably includes two actuators separately used to control flow into each of two hydraulic tines (one for tissue compression and one for stapling). An endoscope 22 in the system is insertable through a lumen in the shaft 16 permits visualization of the plication procedure. The system may optionally include an overtube, such an endoscopic guide tube 23, having a lumen for receiving the stapler 12.

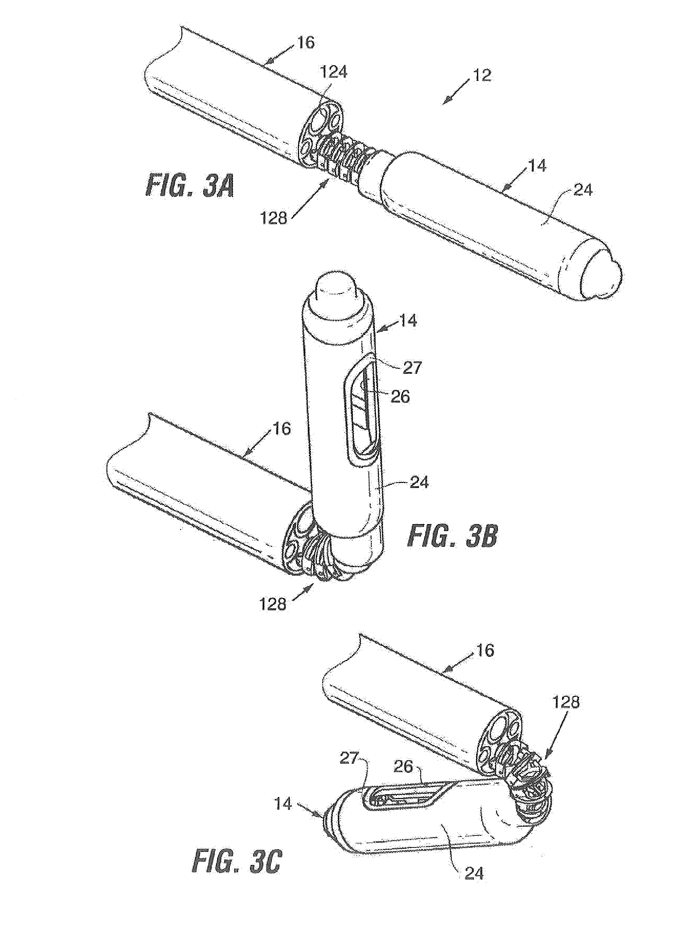

Referring to Fig. 3A, a covering or membrane 24 in the stapler encloses the stapler mechanism to form a vacuum chamber 21 (Figs. 17-23) within the stapler head 14. The side exposed to the tissue to be plicated remains uncovered by the membrane 24 to allow tissue to be drawn into the chamber during use. For example, the membrane 24 may include a side opening 26 as shown in Fig. 3B. Membrane 24 is preferably formed of silicone, elastomeric material, or any other inelastic or elastic flexible or deformable biocompatible material such as a pleated mylar film, capable of forming a vacuum chamber 21 that will expand in volume to accommodate tissue drawn into chamber 21. Also shown in Figs. 3A-3C is an articulating section 128 connecting the instrument shaft to the staple head, and described blow with reference to Figs. 26 and 27.

At least a portion of the membrane is at least partially transparent, In being at least partially transparent, the membrane is formed of a material, or includes sections of material, that will allow the user to see through the membrane well enough to confirm (via endoscopic observation) that an appropriate volume of tissue has been acquired into the stapler head prior to staple application. The opening 26 may be surrounded by a reinforced section 27 formed of material that will strengthen the area around the opening 26. Reinforced section 27 may be formed of a thicker section of the membrane material, and/or a higher durometer material. Alternatively, reinforcing ribs or other structures or elements may be formed into or onto the membrane material, or embedded in the membrane material,

### Stapler Head or Device

The stapler head 14 is designed to have a minimum profile during insertion to the plication site, and to then transform into a much larger profile device having a large internal volume. For example, in one embodiment me vacuum chamber might have an initial internal volume of 0.2 cubic inches, and an expanded volume of 0.6 cubic inches (i.e. the internal chamber volume after subtracting the volume occupied by the stapler head components positioned within the vacuum chamber). This large internal volume allows a large volume of tissue to be drawn into the vacuum chamber and stapled. In this way, the stapler head creates a large plication without requiring invasive techniques for insertion. The unique features of the stapler head allow in situ volumetric expansion of the staple head using a minimum of motion and force input. In particular, as will be seen below with respect to Figs. 32A-C and Fig. 33, the plication can be sized such that the staples applied to the tissue, such that the two annular staple arrays seen in Fig. 33 are well spaced from the edges of the stapled tissue, minimizing the risk of tissue tearing around the staples.

Features of the stapler head are shown in Figs. 4-10. For clarity, the membrane is not shown in these Figures. Referring to Fig. 4, stapler head 14 generally includes a first, staple member 25 comprising a proximal staple housing 28, a second, anvil member 27 comprising a distal anvil housing 30, and at least one elongate member but preferably a pair or hinged arm assemblies 32 that operatively connect the two housing as described below.

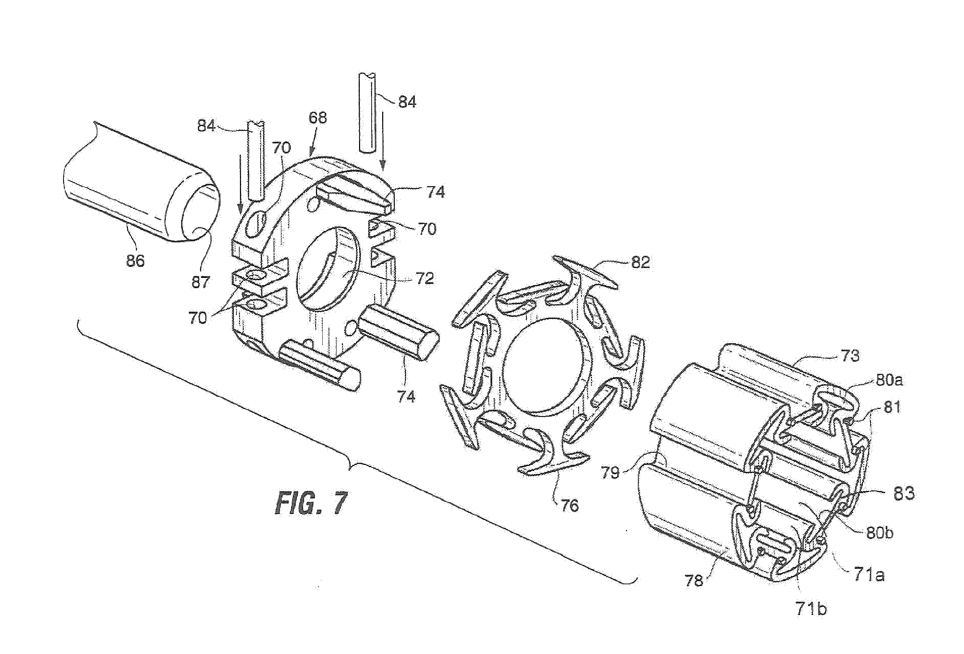

The staple housing and anvil housing are arranged to allow tissue to be compressed between contact surfaces on each of the staple housing and the any housing. In the disclosed embodiment, the contact surfaces are on a staple holding portion of the staple housing, i.e., the outer face of the staple holder, and an anvil on the anvil housing. Considering only the tissue-capture operation of the device, staple holder 78 (shown in Fig. 7) functions as a tissue-capture plate having a front, tissue-contacting surface 83, and anvil 96 ((shown in Figs, 13 and 14) functions as a second tissue-capture plate having a tissue contacting surface. 103 confronting surface 83, where these to surfaces serve to capture the tissue fold during operation of the device, as will be described more detail with respect to Figs. 32A - 32C.

The arm assembles 32 extend between the staple housing 28 and anvil housing 30 on opposite sides of the stapler head 14. Proximal and distal pins 34, 36 pivotally couple each arm assembly 32 to the staple housing 28 and the anvil housing 30. An expansion member comprising a membrane raiser 37 also extends between the staple housing 28 and the anvil housing 30. Although the membrane 24 is not shown in Fig. 4, it should be understood that the membrane raiser 37 is positioned opposite the opening 26 (Fig. 3B) in the membrane. In the illustrated embodiment, membrane raiser 37 includes a link 38 pivotally mounted to the staple housing by a pin 42, a corresponding link 40 pivotally mounted to the anvil housing by pin 44, and spring wires 46 coupling the links 38, 40 to one another.

### Staple Housing

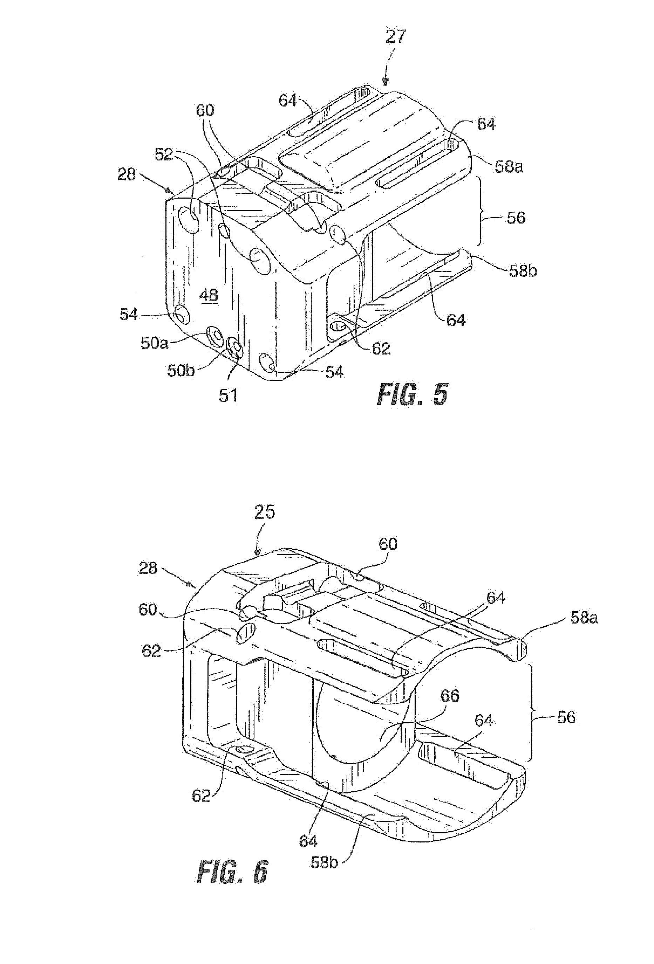

Turning to a more detailed discussion of the stapler head components, the staple lousing 28 can be seen separated from other components in Figs. 5 and 6. As shown in Fig. 5, proximal face 48 of the staple housing includes input ports 50a, 50b through which fluid is directed for hydraulic actuation of the tissue compression, stapling, and optional cutting operations of the stapler head. Seals 51 surround the ports 50a, 50b to minimize fluid leakage.

Vacuum ports 52 are fluidly coupled to a vacuum source 20 (Fig. 2) that is selectively activated to creates negative pressure in the vacuum chamber for tissue acquisition. The vacuum ports 52 are connected to the vacuum source 20 by flexible tubing (not shown) in the stapler shaft 16 (Fig. 2). Mounting holes 54 are used to mount the stapler head 14 to the shaft 16 through the articulating section 128.

The staple housing 28 includes upper and lower sections 58a, 58b above and below open side sections 56. The upper section 58a includes a recess 60 within which the pivot pin 42 for link 38 (Fig. 4) is mounted. As best shown in Fig. 6, bores 62 are positioned in the upper and tower sections 58a, 58b to received pins 34 (Fig. 4) that serve as the proximal pivot points for arm assemblies 32. Guide slots 64 extend longitudinally through the upper and lower sections 58a, 58b.

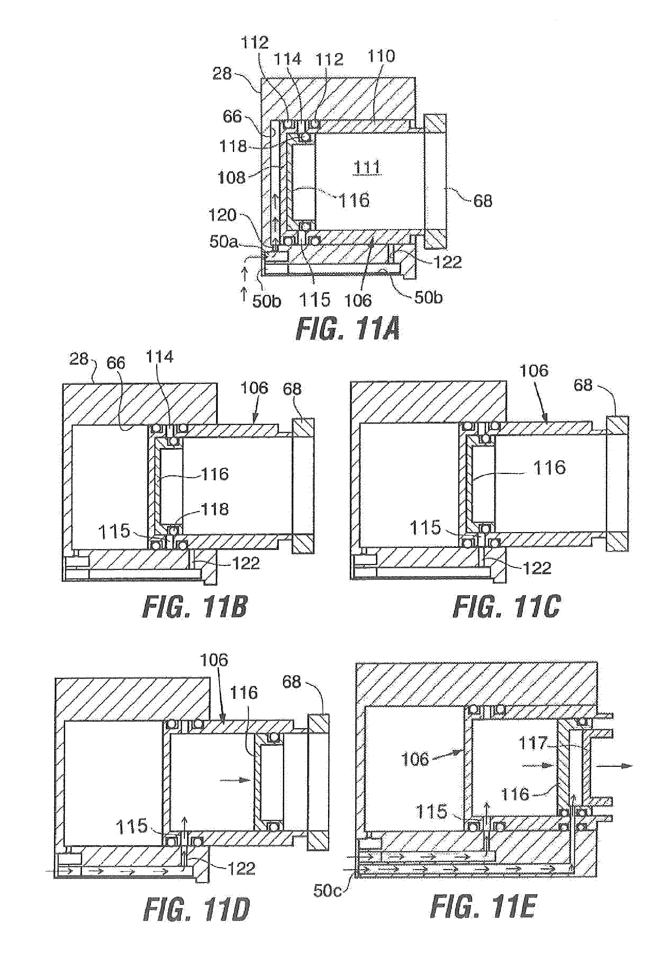

Referring two Fig. 6, a hydraulic chamber 66 is disposed within the staple housing 28. Within the hydraulic chamber 66 (Fig. 6) is a dedicated hydraulic circuit for driving the tissue compression and stapling functions of the stapler, Chamber 66 is fluidly coupled to the fluid input ports 50a, 50b (Fig. 5). As will be discussed in detail in connection with Figs. 11A-11D, fluid driven into the hydraulic chamber 66 via input ports 50a, 50b sequentially advanced a system of hydraulic pistons (not shown) that act on other components to compress the tissue, and that drive the staples and cutting element through the compressed tissue.

Fig. 7 illustrates components of the stapler head that are driven by the hydraulic system for compression, stapling, and cutting. For clarity, these component are shown separated from the staple housing and from each other. In this discussion, the components that are driven by the hydraulic system will be described. The hydraulic system itself is described in a later section in connection with Figs, 11A - 11D.

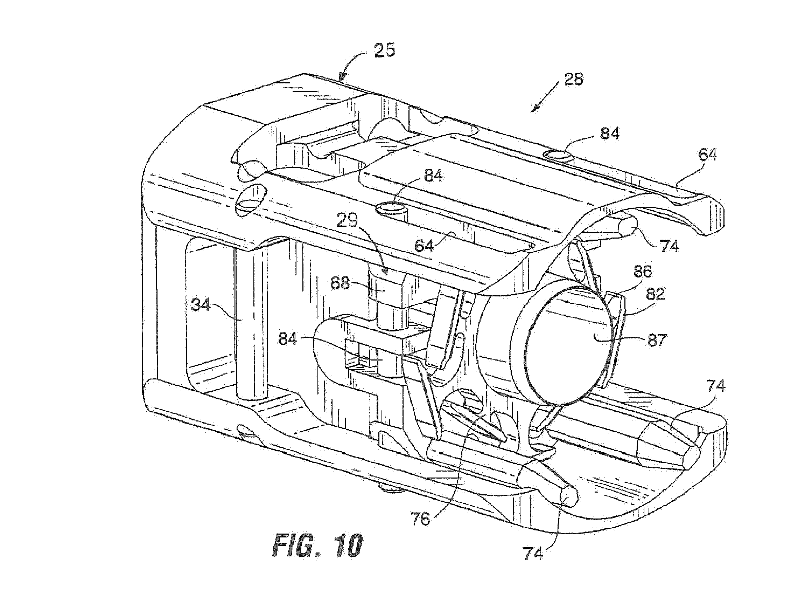

In particular, Fig. 7 illustrates a drive member which takes the form of a disk 68 in the staple housing. In the assembled housing, disk 68 is positioned such that it will be pushed distally by a hydraulic compression piston (piston 106 in Figs. 11A-11E). As will be seen in Figs. 11A-11E, the drive member is movable between a first, retracted position shown in Fig. 11A to a second, extended position shown in Figs. 11C and 11D. Although the drive member illustrated here is driven by, but separate from piston 106, it will be appreciated that these two components can be formed of a single-piece member, i.e., as a single-piece drive member including both piston and disc. As will be seen below, the drive member is coupled to the arm assemblies 32, the anvil housing, and the staple housing so that advancing the drive member distally (toward its extended position) effects tissue compression by bridging the contact surfaces of the staple housing and anvil housing relatively towards one another. The combination of disk 68, its driving piston 106, and assembly arm 32 coupling the two housings is also referred to herein collectively as a drive assembly, indicated at 29 in Fig. 10. The drive assembly may further include drive links 114 in the anvil member, which are operatively linked to assemblies 32 as described below.

As seen best in Fig. 7, disk 68 includes mounting bores 70, a central opening 72, and alignment, posts 74. Referring briefly to Fig. 10, is the assembled stapler head, disk 68 is coupled to the stapler housing 28, and its axial movement therein constrained, by pins 84 that extend through the housing's guide slots 64 and through mounting bores 70 in the disk 68.

A portion of the staple housing 28 contains, i.e., is loaded to contain, staples to be fired into the tissue. The staples are contained within a staple holder, such as staple cartridge 78, on the staple housing. The staple holder may have a number of different configurations. For example, it may be an integral portion of the staple housing, or a separate part mounted or attached to the staple housing, and/or it may be moveable relative to the body of the staple housing to effect tissue compression prior to stapling. In any of these examples, the staple holder may be a removeable/replaceable cartridge, and/or it may be refillable by inserting additional staples into it, In other embodiments, the staple holder may be neither replaceable nor refillable, i.e., i.e., in a dive intended for one-time use.

In the disclosed embodiment, the staple holder is a removeable staple cartridge 78 that can be replaced with another cartridge after staple filing. In this embodiment, the staple cartridge is moveable relative to the body of the staple housing to compress the tissue prior to staple firing.

Referring again to Fig. 7, staple cartridge 78 is positionable within the staple housing, distal to the disk 68, such that distal advancement of the disk by the compression piston pushes the cartridge from a first, retracted position distally to a second, extended position to compress tissue disposed between the cartridge and anvil. Grooves 79 on the exterior of the cartridge slide over corresponding ones of the alignment posts 74 during insertion of the cartridge into the stapler head. Fig. 10 shows the alignment posts prior to loading of a cartridge into the staple housing. As shown, the alignment posts 74 may have tapered ends to facilitate loading of the cartridge over the posts. It will be appreciated that the alignment posts hold the cartridge against angular movement within housing 28 during stapler operation.

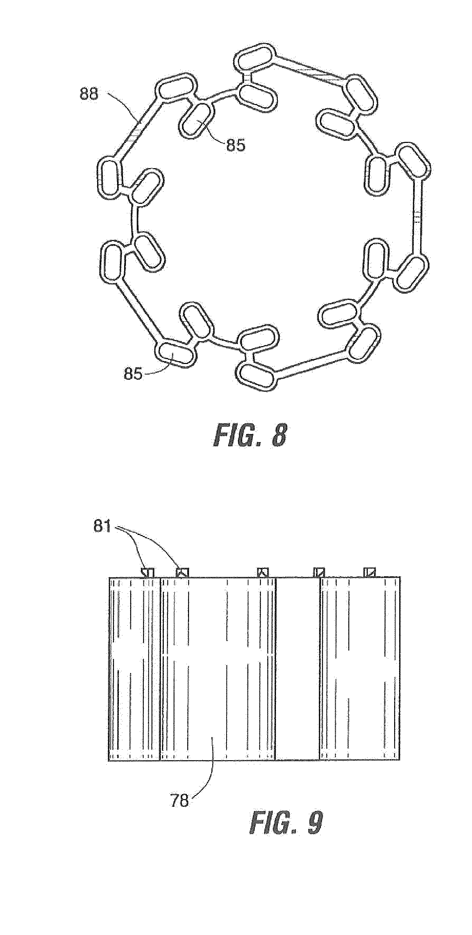

Again referring to Fig. 7, cartridge 78 include a number of staple locations 80, each housing a staple, such as staples 158 seen in Fig. 33. The staple cartridge is equipped with bosses 81 to retain a staple line reinforcement device 83 of the type shown in Fig. 8 and disclosed in detail in commonly-owned U.S. Application No. 11/542,457, entitled ENDOSCOPIC PLICATION DEVICES AND METHODS, filed October 3,2006, and published September 20, 2007 as US 20070219571. To summarize briefly, this type of reinforcement device 83 may be a ring or other element positionable against the distal face of the staple cartridge. When the ring is placed on the cartridge, openings 85 in the ring align with prongs of some of the staples in the cartridge. When staples are driven from the cartridge, these prongs pass through associated ones of the openings 85 and capture the ring 83 against the adjacent body tissue.

Referring to Figs. 7 and 9, a number of undercut bosses 81 on the anvil-facing side of the cartridge may be used to lock the reinforcement device 83 in place on the face of the staple cartridge. Other positive shapes, such as mushrooms, hooks, and tilted bosses could be used to accomplish the same end. Negative shapes, such as pockets or grooves formed into the surface of the cartridge, may also be employed to engage corresponding features on the reinforcement device 83. As another alternative, the reinforcement device may he held in place on the cartridge using adhesives.

In the embodiment shown, a cutter element 86 extends through the central opening 72 (Fig. 7) of the disk 68. The cutter element is shown as a tubular punch having a sharpened wall and a lumen 87, but may be provided in alternative forms. A staple pusher 76 is mounted to the cutter element, distally of the disk as can be seen in the assembled view of Fig. 10. Staple pusher 76 incudes pusher elements 82 proportioned to slide into the cartridge's staple locations 80 as the staple pusher 76 is advanced into the staple cartridge 78, thus driving the staples from the cartridge. A hydraulically-driven staple piston (shown at 116 in Figs. 11A-11E) in the hydraulic chamber 66 (carried within a hydraulic chamber formed by piston 106) is coupled to the cutter element 86 such that advancement of the stapler piston advances the staple pusher 76 and cutter element 86 in a distal direction.

### Fluid Drive System

The fluid drive system used to actuate compression, stapling and cutting may be configured in various ways. The following paragraphs describe one exemplary configuration for the fluid drive system, which in this embodiment is a hydraulic system. Figs. 11A and 11B schematically show the fluid flow in the hydraulic chamber 66 of the staple housing 28 during both compression and stapling stages of actuation. Referring to Fig. 11A, compression piston 106 is disposed within hydraulic chamber 66. Disk 68 (also shown in Figs. 7 and 10) is positioned in contact with or slightly distal to piston 106. Compression piston 106 is generally cup-shaped, having a rear wall 108 and a side wall 110 enclosing an interior 111. O-ring seals 112 are spaced-apart on a proximal portion of the side wall 110. Channels 115 are formed through the side wall 110, between the o-ring seals 112.

A second piston, referred to as the staple piston 116, is positioned in the interior 111 of compression piston 106, against the rear wall 108. Although not shown in Figs. 11A-11D, cutting element 86 (Fig. 7), with the staple pusher 76 thereon, is positioned in contact with or slightly distal to the staple piston 116. An o-ring seal 118 Surrounds a portion of the staple piston 116 that is distal to the channels 115 in the compression piston.

A first fluid channel 120 extends from fluid port 50a in the stapler housing 28 to a proximal section of the hydraulic chamber 66. A second fluid channel 122 extends from fluid port 50b in the stapler housing to a more distal section of the hydraulic chamber 66. Fluid flow from port 50a and fluid channel 120 against the compression piston cylinder is shown in Fig. 11A. Fluid pressure within the hydraulic chamber 66 advances the compression piston 106, with the stapler piston 116 within in it, in a distal direction, from a first, retracted position, shown in Fig. 11A to a second, extended position shown in Figs. 11C and 11D. Fig. 11B shows the compression piston 106 approaching the end of its travel, i.e., fully extended position. Once the compression piston reaches the end of its travel as shown in Fig. 11C, channel 115 in the compression piston 106 aligns with channel 122 in the housing, allowing fluid introduced through fluid port 50b to enter the interior of the compression piston 106 via channel 122. The fluid entering the interior of the compression piston drives the staple piston distally as shown in Fig. 11D, from a first, retracted position shown in Figs 11A- 11C, to a second, extended position shown in Fig. 11D. In an alternative embodiment shown in Fig. 11E, a third piston 117 is provided for separately driving the cutting element 86. In this embodiment, fluid introduced into a third drive fluid port 50c causes advancement of the third piston 117 from a first, retracted position to a second extended position (not shown). The pistons 106, 116 and 117 and associated fluid paths may be arranged so that fluid cannot enter the interior of the stapler piston to advance the cutting piston 117 until compression piston 106 has traveled to the tissue-compression position and stapler piston 116 has in turn traveled to the stapling position.

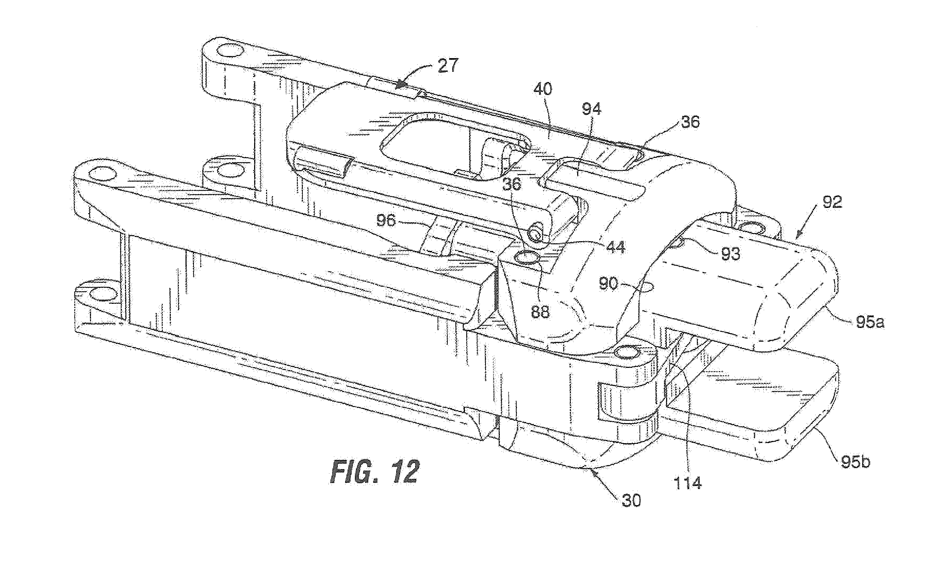

The anvil housing (identified by numeral 30 in Fig. 4) in anvil member 27 will next be described with reference to Fig. 12. The anvil housing 30 includes mounting bores 88 for receiving pivot pins 36 at the distal end of the hinged arm assemblies 32. The upper section of the anvil housing 30 includes a section 94 through which the pivot pin 44 for link 40 (Fig. 4) is mounted.

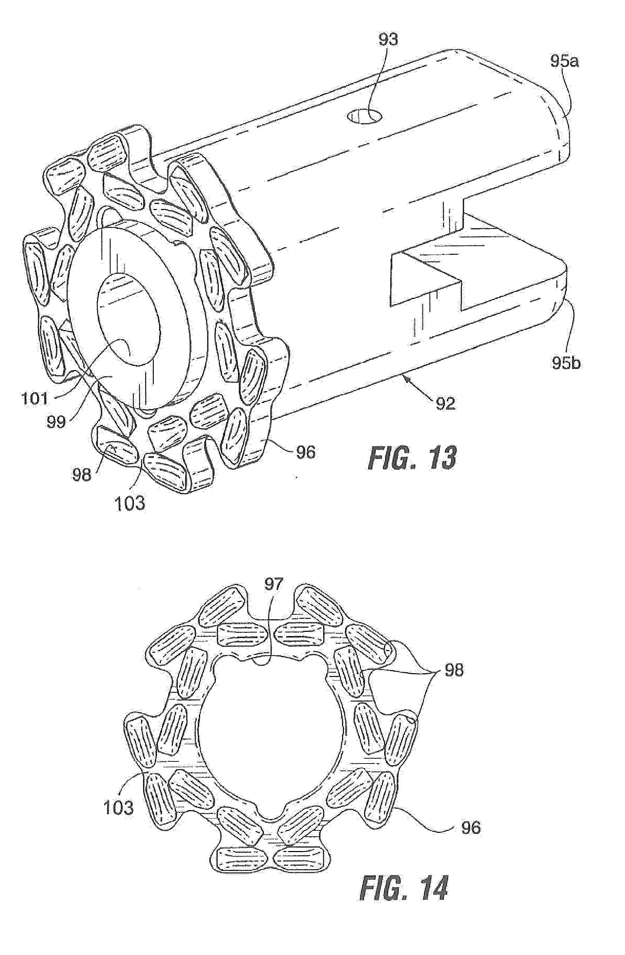

A central bore 90 extends longitudinally through the anvil housing 30. An anvil support 92 (Fig. 13) is longitudinally slidable within the bore. Both the bore 90 and the anvil support 92 are preferable formed to have non-circular cross-sections (such as the illustrated rectangular cross-section) with flat bearing surfaces to prevent rotation of the piston within the bore.

Fig. 13 shows the anvil support 92 separated from the anvil housing 30. The distal portion of the anvil support 92 is split into upper and lower plates 95a, b. Plate 95a has a bore 93 axially aligned with a similar bore in plate 95b. The proximal portion of the anvil support 92 carries the anvil 96. As shown in Fig. 14, anvil 96 includes plurality of indentations 98 positioned such when staples are driven from the staple cartridge, each staple leg engages one of the indentations, which causes the staple leg to fold or crimp, In the embodiment shown, the anvil is designed for a staple array having two annular rings of offset staples, five staples per ring. A central opening 97 extends through the anvil 96 and is contiguous with a lumen in the anvil support 92.

The anvil 96 and the staple cartridge 78 (Fig. 7) are the two parts of the stapler head which exert force on the tissue to be stapled. As shown in Figs. 9 and 14, the preferred anvil and cartridge are designed to use a minimal amount of material surrounding the indentations 98 of the anvil 96 and the staple locations 80 of the cartridge 78 - so that the amount of anvil/cartridge surface area-contacting the tissue is as small as possible. When subjected to a constant force, a smatter footprint will damage less tissue than would a larger footprint, since to smaller area of tissue is squeezed between the anvil and cartridge. However, the tissue that does get squeezed experiences more pressure from the given force because the force is distributed over a smaller area. In other words, the minimized footprint creates more pressure on the tissue with less force. This is advantageous from a mechanical standpoint because the stapler head need not supply or withstand as much force as would be needed with a larger-footprint cartridge and anvil.

Referring to Fig. 7, in the illustrated embodiments, the staple cartridge 78 has an outer wall that tracks the contours of the staples housed within it, thus forming a number of pedals 73 surrounding the outer staple positions or slots 80a. with the grooves 79 disposed between the pedals, adjacent to the inner staple positions 80b. Rather than providing each staple position to be fully surrounded by cartridge material, the staple positions 80a, 80b preferably each include a back waff 71a and a retaining element attached to the wall and positioned to retain a staple between the retaining element and the back wall. In Fig. 7, the retaining element comprises a pair of wings 71 b that curve inwardly from the back wall 71 to define a slot that is sufficiently bounded to retain a staple within the staple position, but that is preferably not bounded around its full circumference. The anvil has a simitar pedal arrangement, as shown in Fig. 13.

Referring again to Fig. 13, a plate 99 is positioned on the anvil 96 such that the distally-advaticing cutting element 86 will advance into contact with the plate 99 during tissue cutting. In one embodiment, the plate 99 may be seated within the opening 97 in the anvil. The plate 99, which will also be referred to as the "cutting board", has a hole 101 in it which relieves the pressure of the captured tissue and prevents hydraulic locking, a condition in which the punch and plate create a closed volume, If it is desired to move the cutting element 86 after contact is made, pressure will increase inside this closed volume and it will resist further motion. This may prevent or adversely affect tissue cutting,

The cutting board is preferably designed so as to not serve as a hard stop against advancement of the cutting element 86. If the cutting element 86 is stopped by the cutting board, the stapling piston will also be stopped and incomplete staple formation may result. Therefore, it is preferred that the cutting element 86 is allowed to penetrate or displace the cutting board during and after the tissue is cut; that is the cutter can advance slightly once initial contact with the board is made.

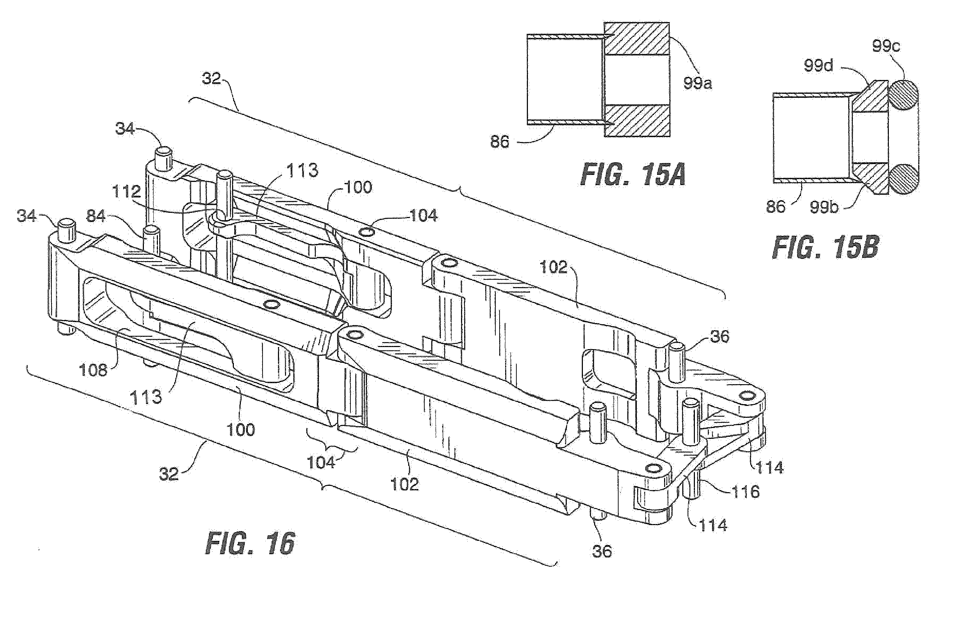

Figs. 15A and 15B illustrate the cutting element 86 advanced into contact with differed embodiments of cutting boards. In the Fig. 15A embodiment, the material of cutting hoard 99a is a relatively soft material, such as an elastomeric silicone, which is cut or penetrated by the advancing cutting element as shown. This material allows the sharp distal end of the cutting element to move into the cutting board during the final stage of staple formation. In the Fig. 15B embodiment, the cutting board 99b can be made of a harder material positioned with a compressible object such as an elastomeric spring 99c behind it. In the figure, this spring is an o-ring. Advancement of the cutting element 86 against the cutting board 99b causes the cutting board to be displaced distally against the spring 99c. The advancing cutting element 86 experiences increasing resistance as the o-ring is compressed. Other spring shapes and materials, such as coiled wire, spring washers and leaf springs can be used to achieve the same result. The chamfer 99d on the surface of the cutting board 99b may help to align the cutting element 86 as it is forced into contact with the cutting board.

### Arm Assemblies

Following is a discussion of the features of the arm assembles 32. Fig. 16 shows the arm assemblies 32 separated from the other elements of the stapler head. In general, each arm assembly has a first arm section 100 pivotally coupled to the staple housing and a second arm section 102 pivotally coupled between the first arm section and the anvil housing. While not present in the illustrated embodiment, additional arm sections may be positioned between the first and second arm sections.

That is, each arm assembly includes a proximal arm 100 and a distal arm 102 joined to one another to form a hinge 104. Each of the proximal arms 100 has a longitudinal cutout 108 and an arm spreader 113 pivotally mounted within the cutout 108. The distal end of each arm spreader 113 includes a bore 112. Pin 84 is positioned within the bore 112. As disclosed in connection with Fig. 10, this pin 84 extends through the disk 68 and has ends that ride within the slots 64 (Fig. 6) on the lower and upper sections of the stapler housing. Longitudinal movement of the disk 68 within the stapler housing will thus advance the pins 84 within their corresponding slots 64, causing the arm spreaders 113 to pivot relative to die pins 84 and to thus drive the arm assemblies 32 outwardly. Additional specifics concerning movement of the arm assemblies 32 is set forth in the section entitled Stapler Head Operation.

Distal arms 102 of the arm assemblies include pins 36 which, as discussed, are pivotally mounted to the anvil housing 30 (Fig. 4). A pair of drive links 114 are provided, each of which has a first end pivotally attached to a corresponding one the distal arms 102 and a second end pivotally coupled to a common pin 116. In the assembled. Stapler head, pin 116 is positioned in the bores 93 of the upper and lower plates 95a, 95b of the anvil support (sec prates 95a, b in Fig. 12). As detailed in the Stapler Head Operation section below, when the arm spreaders 113 drive the arm assemblies 32 outwardly, drive links 114 act on the pin 116 to push the anvil support in a proximal direction, causing the anvil to advance proximally towards the staple cartridge.

Alternatively, the anvil may be driven within its housing independently of the arm assemblies, by a direct hydraulic drive mechanism carried in the anvil housing. Preferably, the drive mechanisms in the two housings are coordinated so that the staple holder and anvil are moved toward one another in their respective housings at the same time.

### Stapler Head Operation

The following discussion centers on the manner in which the arm assemblies function to expand the vacuum chamber and to compress tissue that has been drawn into the chamber using suction. As an initial step preceding chamber expansion, the stapler head is positioned with the opening 26 in the membrane 24 in contact with tissue at the location at which plication creation is desired. Vacuum source 20 (Fig. 2) is activated to apply vacuum to the inside of the vacuum chamber defined by the membrane. Tissue in contact with the opening 26 (Fig. 3B) will be drawn into the vacuum chamber between the staple housing 28 and the anvil housing 30. After the tissue is drawn in, the stapler profile is changed, expanding the volume of the chamber within the membrane.

The streamlined position of the stapler head 28 prior to expansion is shown in Figs, 4, 17 and 18. In particular, the hinged arm assemblies 32 and membrane misers 37 are in generally straight orientations. The proximal arms 100,serve, as the drive arms for chamber expansion and tissue compression. Motion of these arms is initiated when water under pressure is forced into the hydraulic circuit of the staple housing. Referring to Fig. 19, the fluid pressure advances disk 68 (by action of the compression piston 106, not shown in Fig. 19). Disk 68 in turn pushes the staple cartridge 78 toward the anvil 96 as shown in Figs. 19-21, causing the staple cartridge 78 to extend further from the staple housing 28.

Both the disk 68 and the arm spreaders 113 are coupled to the pins 84. For this reason, the longitudinal movement of the disk 68 within the stapler housing 28 will carry the pins 84 distally within their corresponding slots 64. The arm spreaders 113 will consequently pivot relative to the pins 84, driving the proximal arms 100 outwardly. Outward movement of proximal arms 100 at hinge 104 causes the distal arms 102 to also pivot outwardly at hinge 104, forming an angle between the proximal and distal arms 100, 102. Naturally, formation of the angle between the arms 100, 102 shortens the effective length between the remote ends of the arms, causing the distal pins 36 of the distal arms 102 to carry the anvil housing 30 towards the staple cartridge. The pivoting movement of the distal arms 102 further causes drive links 114 to act on pin 116 to push the anvil support in a proximal direction. This moves the anvil support relative to the anvil housing in a proximal direction at the same time the anvil housing is also moving proximally.

In essence, one motion, that of the hydraulically driven compression piston, creates at least three motions, illustrated by arrows A1, A2 and A3 in Figs. 19-21. These three motions include: the staple cartridge 78 moving relative to the staple housing in a direction towards the anvil 96 (arrow A1), the anvil housing 30 moving toward the staple housing 28 (arrow A2) and the anvil 96 itself moving relative to the anvil housing 30 in a direction towards the cartridge (arrow A3). This compound motion of the anvil toward the staple cartridge enables a small displacement of the compression piston to quickly compress tissue in the grip of stapler. The multiplication of motion also enhances force transmission between the two housings by keeping the angle at hinge 104, between the proximal (driven) arm and the distal (drive) arm, as large as possible.

The relative motion of the two housings 28, 30 toward each other also drives upward links 38, 40 and their interconnecting spring wires 46 on the top of the stapler head 14. Together, the links and spring wires raise the top of the membrane, creating more volume to accommodate expansion of the tissue during compression.

Compression of the tissue is halted when the pins 84 traveling in slots 64 in the staple housing 28 reach the limit of travel, as shown in Figs. 22 - 24. Thus, the slots and associated components are dimensioned to set the desired separation distance between the tissue contact, surfaces on the stapler side and the anvil side of the stapler head. Exemplary separation distances for use in stomach wall plications might include approximately 0.06-0.07 inches (e.g. for use with staples having legs of 5.5mm length) or 0.109 inches for 6.5mm leg length staples. Application of additional pressure into the hydraulic circuit will not compress the tissue any further.

Moreover, because of the piston arrangement, the stapling function is effectively locked out until tissue compression is complete. With this arrangement, fluid introduced via the fluid port 50b (Fig. 11A) into the staple fluid channel 122 prior to completion of tissue compression will leak until the two o-rings 112 of the compression piston 106 are straddling the inlet 114. This design prevents premature staple firing.

At the fully compressed position, the arm spreaders 113 are nearly perpendicular to the longitudinal centerline of the stapler head. Once tissue is compressed between cartridge 78 and anvil 96, the tissue is ready for stapling.

Stapling is initiated by introducing hydraulic fluid through port 50b (Fig. 5). The staple piston advances, pushing cutting element 86 (Figs. 7 and 10) towards the anvil 96. Because the staple pusher 76 is mounted to the cutter 86, this action carries the staple pusher 76 through the cartridge 78 where it simultaneously pushes all staples through the tissue. Staple piston travel is limited by internal stops, and is preset to yield optimal staple formation.

During compression, as the angle at the hinge 104 of arm assemblies 32 reaches its minimum, the forces required to resist separation of the staple and anvil housings increases. These forces increase further when the forces of staple crushing are exerted on the anvil by the staple piston. To compensate, the arm spreaders 113 serve as displacement struts to channel at least a portion of these forces into the disk 68. These forces, if not reacted by the pusher disk, would pull in the arms 100, 102 and potentially release the compression on the tissue, causing incomplete staple formation or tissue cutting. In this way, a truss-like structure is created for force displacement.

When staples have been formed, staple pressure is released and a spring (not shown) returns the staple pusher 72 to its base position. Releasing fluid pressure will allow the deflected spring wires 46 on membrane raiser 37 to return the staple head to its minimum profile configuration and release the plication from the stapler. Once outside the patient, the used staple cartridge can be ejected and a new one installed.

Figs. 25A - 25C illustrate one method for, retaining a removable staple cartridge 78 within the staple housing. The cartridge is spring-loaded into the staple housing and retained by two latches 170 (one visible), each pivotable relative to a fulcrum 172. As shown, the fulcrum 172 may be coupled to the disk 68 by pin 84. Each latch 170 includes a catch 174 which engages a corresponding catch 176 on the cartridge. The latch 170 is preferably spring biased to urge the catch 174 inwardly towards the cartridge.

Depressing the proximal end 175 of each latch 170 as shown by arrow P in Fig. 25B pivots the latch against this bias, causing ejection of the staple cartridge. A new staple cartridge may then be positioned with its grooves 79 aligned with alignment posts 74 as shown in Fig. 25C and then pushed towards the staple housing. As the now cartridge slides into position, catch 174 rides over the tapered proximal portion 178 of the catch 176. Once catch 174 passes over the distal end 180 of the catch 176, it drops inwardly towards the cartridge due to its spring bias, thus engaging the cartridge. When the cartridge is properly seated, a click will be felt or heard as the latches engage the new cartridge.

### Stapler alignment during stapling

In operation, as the two stapler members are brought together, a tissue fold is captured between the outer face of the staple cartridge and the confronting face of the anvil. As this tissue capture is occurring, as just prior to and during the stapling of the tissue fold, variations in the thickness and or compressibility of regions of the captured tissue may bias the stapling operation off-center, i.e., off axis, causing the staple legs to be received out of position within their associated anvil indentations, with the result that one or more of the staples may fastened defectively, e.g., without complete folding of the staple legs, and/or the array of staples may be offset with respect to the center axis of the device, causing some staples, for example, to be too close to the hole cut in the center of the stapled fold,

The insure that the stapling operations occurs successfully for each staple array in the cartridge (for example, two concentric arrays of five staples each), the device of the invention may include alignment structure to hold to proximal and distal members of the device in axial alignment just prior to and during the stapling operation. The alignment structure, is shown in side sectional views in Figs, 36 and 37. Shown in Fig. 36 is the portion of the staple device 12 that includes anvil 96 and cutting board 99a on distal member 27, and cutter 86 on proximal member 25. In operation, the two members, and the staple cartridge and anvil carried thereon, are first moved together, to capture a tissue fold between the confronting faces of the cartridge and anvil, as illustrated in Figs. 22 and 23. Staple piston 116 is then moved from its retracted to its extended position, as seen in Fig. 24, to move the cutter against cutting board 99a, as shown, and drive the staples in the staple cartridge against the anvil.

The alignment structure includes a pin held axially within cutter 86 for movement therewith, and a pin-receiving slot formed in a bushing 164 supported within a a portion of housing 27 attached to anvil 96 for axial movement therewith. In the embodiment shown in Fig. 36, bushing 164 is spring loaded, through a spring 166, within housing 27 in a a direction that resists movement of the pin toward the bushing, allowing the cutter and attached pin, which has now penetrated through the captured tissue, to seat in the beveled end of the bushing. As the cutter, pin, and staple pusher continue to move toward the second member, ultimately forming a hole in the tissue and ejecting staples from the staple cartridge through the tissue and against the anvil, the two device members are maintained in axial alignment, so that the stapling operation occurs with all staples positioned in alignment with the respective to the associated anvil indentations. When this operation is complete, the staple piston and drive member are retracted to release the stapled tissue and return the device to its linear condition.

The embodiment of device 12 shown in Fig. 37 is similar to that just described, but where the alignment pin 168 carried in the housing of cutter is spring biased, by a spring 174, in the direction of the second member, and a pin-receiving slot 170 is in a fixed-position bushing 172 carried below the anvil (not shown) in member 27. In this embodiment, motion of the two members toward one another, once the pin has been initially seated in the bushing slot, is accommodated by movement of the pin in a direction away from the second member, as the staple piston continues to move toward its fully extended position, to cut and staple the tissue captured between the two members, as described above.

### Stapler Shaft and Handle

Referring again to Fig. 2, the stapler shaft 16 connecting the handle 18 and the stapler head 14 is flexible enough to conform to the curvature of the upper digestive tract, yet maintains the ability to transmit enough torque to rotate the stapler head. The shaft is formed with sufficient stiffness to allow it to be pushed down esophageal guide tube 23. Suitably materials include

Fig. 26 shows a distil portion of the shaft 16, with the stapler head removed from the shaft. As shown, shaft 16 includes an endoscope lumen 124 through which an endoscope is advanced to allow visualization of a stapling operation. Side lumens 126 may also be provided for receiving other instruments useful during the procedure.

An articulating section 128 is positioned at the distal end of the shaft 16, between the shaft 16 and the stapler head 14 so as to allow the stapler head to be articulated relative to the shaft. Tubing coupled to the vacuum source and the source of hydraulic fluid extends from the handle and through the shaft 16 and the articulating section 128.

Fig. 27A shows one configuration that may be used for the hydraulic fluid lines 130. During use, the hydraulic fluid lines are subjected to significant deflection and elongation in the articulating section of the stapler. They are also subjected at times to fluid pressure which may be in excess of 1,500 psi. Typically, hydraulic lines in industrial applications are flexible and have a working loop of extra tubing that accommodates length changes during use. The illustrated configuration for the hydraulic lines is a tower profile solution particularly suitable for an endoscopic device having space constraints. A preferred hydraulic line is a tube 130 having a portion that is shaped into a longitudinally expendable shape so that it can accommodate effective length changes during banding. The longitudinally expandable portion of the tube is preferably disposed within the articulating section 128 of the stapler 12. In a preferred design, the longitudinally expandable shape is a coil shape as shown in Fig. 27A. In alternate embodiments, the tube 130 may be formed into other longitudinally expandable shapes, such as regular or irregular undulating shapes (Fig. 27B).

The preferred material for the tubes 130 is stainless steel hypotube, although other materials may instead be used. In the preferred stapler configuration, two drive fluid lines are provided, one for actuating tissue compression, and the other for staple application (and cutting when used), In the present embodiment, the tubes are coiled together as shown in Fig. 27A. In alternate embodiments, two or more coiled tubes may be nested one inside the other. As the articulating section bends, it forces the coiled tubes 130 to bend and to change length in response to bending. The coiled tubes behave just as coiled wires would during these motions and are thus able to change length, deflect, and follow the contour of the articulating section without compromising flow through the lumens of the tubes or imparting undue stress to the connection at either end of the hydraulic system.

The longitudinally expandable shapes for the fluid lines may be suitable for use in allowing delivery of fluid to the operative ends of other types of articulating medical devices, such as catheters or endoscopic devices for delivering therapeutic agents or irrigation fluids past all articulating or bendable section of the device.

Referring again to Pig. 26, articulating section 128 is comprised of a spine formed of a plurality of links 132 strung over a pair of pull cables 134 (only one shown in Fig. 26). In one embodiment, engagement of the pull cables allows the stapler head 14 to be articulated in two directions through a range of motion of approximately 90 degrees in one direction (see Fig. 3B) to 175 degrees in the opposite direction (see Fig. 3C). Each pull cable is anchored at or near the stapler head, such as at the distalmost link 132 of the stapler housing 28.

The more proximal portions of the pull cables 134 extend the length of the shaft 16 and terminate in the handle 18. Referring to Fig. 28, the handle 18 includes a rotating knob 136 that may be selectively rotated in a clockwise or counterclockwise to articulate the stapler head up or down. Rotation in one direction applies tension to one of the pull cables to cause the stapler head to bend downwardly, whereas rotation in the opposite direction puts tension on the other cable, causing the head to bend upwardly.

In a preferred handle configuration, the knob 136 includes an internal threaded bore 138. Knob 136 is partially restrained within the handle 18 so that it remains fixed within the handle but can rotate freely. A carriage 140 having a threaded exterior surface is positioned within the threaded bore 128 of the knob. The threads within the bore 138 are engaged with the threads on the carriage 140 so that rotation of the knob causes the carriage 140 to translate, but not rotate, within the handle.

Each of the two pull cables, identified in Fig. 28 as cables 134a and 134b, is terminated on a different member in the handle. Cable 134a is mounted on the sliding carriage and cable 134b is mounted to a stationary part of the handle 18. Each cable extends through a corresponding sheath. Cable 134a extends through a sheath 135a having a proximal end fixed to a stationary part of the handle 18. Cable 134b extends through a sheath 135b having a proximal end mounted to the sliding carriage.

The cables 134a,b and sheaths 135a,b are arranged such that translation of the carriage in one direction will cause deflection of the stapler head in one direction, and translation of the marriage on the other direction will deflect the stapler head in another direction.

Referring to Fig. 28, if knob 136 is rotated to causes the carriage 140 to translate to the left of the page, cable 134a will be tensioned and cable 134b will slacken, causing the stapler head to articulate in a first direction (e.g. upwardly). Rotation of the knob 136 in the opposite direction will advance the carriage to the right of the page, releasing tension on cable 134a and pushing sheath 135b over the cable 134b towards the distal end of the staple head, causing articulation in the second direction (e.g. downwardly) as the sheath 135b is advanced against a distal portion of the shaft 16. The proximal portion of sheath 135b is provided with sufficient working length prevent it from being placed under tension when the carriage moves distally. The positioning of the knob is advantageous in that the hand movement required for stapler articulation is always the same, regardless of the rotational orientation of the stapler. Also, the use of the threaded knob can prevent unintentional relaxation of the deflection angle, even if the knob is provided without a lock to retain its rotational position,

Referring to Figs. 28 and 29, the endoscope lumen 124 extends along the center axis of the stapler. The positioning of the lumen and the coaxial relationship of the articulation knob in relative to the endoscope 124 allows the endoscope and stapler to be rotated independently without one interfering with one another. Thus, if the user chooses to change the rotational orientation of the stapler head 14 within the body, s/he may rotate the handle 18 and shaft 16 while maintaining the rotational position of the endoscope.

For cost efficiency, the stapler 12 may be designed to permit the stapler head 14 to be discarded while allowing the shaft 16 and handle 18 to be sterilized and re-used, One mechanism for removably coupling the stapler head to the shaft 16 is illustrated although others are readily conceivable (e.g. a slip coupling type arrangement). Referring to Fig. 26, an end plate 142 is mounted to the distalmost one of the links 132. Each of the end plate 142 and the corresponding rear surface of the stapler head are provided with latch features that allow the end plate and stapler head to be engaged to one another.

End plate 142 includes a cantilevered pin 144 having a peg 145 (which may be a spring pin), a central opening 146, and a pair of u-shaped catches or engagement edges 148 along its edges. Hydraulic feed holes or openings 156a, b are formed through the end plate 142. The hydraulic tubes that deliver hydraulic fluid to the stapler head (see tubes 130 of Fig. 27) are preferably welded to the end plate to allow fluid from the tubes to be directed through the feed holes 156a, b.

Figs. 30A and 30B show the rear surface 48a of the staple housing, which has been somewhat modified relative to Fig. 5. In this variation of the rear surface 48a, the hydraulic input ports or openings 50a, 50b are repositioned as shown. Additionally, the rear surface 48a. has been modified to include a pair of catches or engagement edges in the form of undercut bosses 150, plus an aligning pin 152, and a hole 154.The snap-on connection of shaft to stapler device;

Figs. 30A and 30B show the end plate 142 positioned against the rear surface 48a of the staple housing. The other features of the articulating section 1 28 are not shown in Figs. 30A and 30B for clarity. To attach the stapler head to the shaft 16, the plate 142, attached to the handle assembly, is pressed against the rear surface 48a of the staple housing as shown in Fig. 30A. As the plate is pushed, it is rotated in a clockwise direction, causing the peg 145 (Fig. 26) of the cantilevered pin 144 to engage hole. 154 in the rear surface of the staple housing. When this latch is engaged, hydraulic feed holes 156a, b of the end plate 142 are lined up with the hydraulic inlets 50a, 50b on the staple head as shown in Fig. 30B. At the same time, portions of the end plate surrounding u-shaped catches 148 slide beneath the undercut bosses 152. Pressing the plate compresses the face-sealing o-rings surrounding the hydraulic input ports 50a, 50b. Compression on the o-rings is maintained by engagement of the catches and the undercut bosses overhanging the end plate. To remove the stapler head from the housing, the stapler housing is twisted in a counterclockwise direction to disengage the end plate 142 from the rear surface 48a. The stapler shaft and handle may then be sterilized in preparation for mounting of a fresh stapler head.

### Exemplary Procedure

One example of a method for using the system 10 will next be described in the context, of formation of plications in stomach wall tissue, with particular reference to Figs. 31-33.

As an initial step (Fig. 2), endoscopic guide tube 23 is advanced into the stomach via the mouth and esophagus. The endoscope 22 is inserted into the endoscope channel in the stapler handle (not shown) and advanced down the lumen of the stapler handle. The stapler/endoscope are simultaneously passed through the endoscopic guide tube towards the stomach. Once the stapler and endoscope reach the gastroesophageal junction region of the stomach, the position of the stapler is maintained white the endoscope is advance further into the stomach.

The stapler head 14 is advanced to the desired depth and location in the stomach. Using the articulation controls on the stapler handle, the angular orientation of the stapler head is adjusted to allow positioning of the stapler head 12 at the pre-identificd target tissue as shown in Fig. 31A. The opening 26 in the membrane 24 is positioned against the target tissue. The endoscope 22 is placed in a retroflexed position as shown.

The vacuum source 20 (Fig. 2) is coupled to the vacuum port on the handle external to the body, and vacuum pressure is applied to draw tissue 17 through the opening 26 and into the vacuum chamber defined by membrane 24 as shown in Figs. 31B and 32A. Acquisition of the target tissue will be readily identified endoscopically through the wall of transparent membrane 24 on the stapler head.

The fluid source (is shown) is coupled to the handle. Once it has been visually confirmed that a sufficient amount of tissue has been acquired, fluid is introduced to cause compression of the tissue and expansion of the arm assemblies 32 and membrane raiser 37 as shown in Figs. 32B and 31C. As can been seen, the expansion of the arm assemblies and the membrane allows a large volume of tissue to be acquired into the vacuum chamber and displaced further into the chamber during tissue compression. As noted earlier, the drawing in of tissue into the expanded chamber during operation, well "above" the staple holder and anvil in Figs. 32B and 32C, provides a relatively large margin of tissue around the stapled portion of the tissue, reducing the risk of tissue tearing or tissue-fold weakness near the stapled portion of the tissue. The captured tissue fold is indicated at 17a.

Once the tissue has been compressed, additional hydraulic fluid is introduced to cause slapling and cutting of the tissue as shown in Figs. 31D and 32C, forming a plication P, indicated at 17b in Fig. 33. The compression and stapling hydraulic sources are then deactivated to release fluid pressure within the hydraulic circuit. With the hydraulic pressure relieved, the spring wires of the membrane raiser 37 help to restore the stapler head 14 to its original streamlined configuration, allowing the stapler head to be withdrawn from the tissue as shown in Fig. 31E. The stapler head may be articulated relative to the shaft to assist in moving the stapler head away from the plication P.

In a preferred plication configuration shown in Fig. 33 the staples 158 are arranged in two concentric rings of five staples, with the staple reinforcement device 83 retained by the staples and distributing forces around the staple patter as shown. The plication P includes a hole H formed by the cutting element, through which various implants or anchors for various implants can be placed.

If multiple plications are needed, the stapler 12 is briefly withdrawn from the endoscopic guide tube and the staple cartridge is replaced in the manner described in connection with Figs. 25A - 25C. The procedure is repeated until all desired plications have been formed.

The systems may be packaged with instructions for use instructing the user to use the various disclosed features to perform a stapling procedure using methods disclosed herein.

### Alternate Embodiments

The basic architecture of the stapler disclosed above can be used as a foundation for other stapling tools, Figs. 34-35 show a modified stapler in which the membrane and membrane- raiser have been removed, and in which the staple housing 28 has been modified for the attachment of tools. As shown in Fig. 34, the staple housing 28 includes a pair of grooves 160 proportioned to receive tools 162. Tools 162 may be seated in these grooves 160 and mounted to the staple housing as shown in Fig. 35. This attachment will provide for a stable base from which to actuate the tools. The tools may be self-articulating, or the staple housing 28 may be equipped with devices 164 for moving the tools between streamlined positions for insertion of the assembly into a body cavity, and a deployed position such as that shown in Fig, 35. Tools similar to those in Fig. 35 might be used for tissue acquisition, by reaching between the cartridge and anvil and used to engage tissue and pull the tissue into position between the cartridge and anvil so that it may be stapled, or otherwise affected by various features added to or in place of the anvil and cartridge. Procedures which may benefit from adaptation of the stapler include, but are not limited to gastroplasty, stoma adjustment, polyectomy, lead placement, bleeding control, perforation or hole closure, biopsy and tumor removal.

The disclosed systems provide convenient embodiments for carrying out the disclosed compression and stapling functions. However, there are many other widely varying instruments or systems may alternatively be used within the scope of the present invention, Moreover, features of the disclosed embodiments may be combined with one another and with other features in varying ways to produce additional embodiments. Thus, the embodiment described herein should be treated as representative examples of systems useful for forming endoscopic tissue plications, and should not be used to limit the scope of the claimed invention.

Any and all patents, patent application and printed publications referred to above, including those relied upon for purposes of priority, are incorporated herein by reference.

Embodiments describe hereinbefore in detail are defined by the following numbered paragraphs:
1. A stapler device (14) for applying a staple to a tissue, comprising,
   a staple member (25) having a staple housing (28) and a staple holder (78) that is independently movable with respect to the staple housing,
   an anvil member (27) having an anvil housing (30) and an anvil (96) carried on the anvil housing (30), and
   a drive assembly (29) including a drive member (68) operatively connected to the staple holder (78) for movement within the staple housing (28) from a retracted position to an extended position, and an arm assembly (32) operatively coupled to the staple and anvil members (25, 27), such that movement of the drive member (68) from its retracted to its extended position is effective to (i) move the staple holder with respect to the staple housing (78) toward the anvil (96) (A1), and (ii) move the anvil member (27) toward the staple member (A2).
2. The device of paragraph 1, wherein the anvil (96) is independently movable in the anvil housing (30), and the arm assembly (32) is operatively coupled to the anvil (96), such that movement of the drive member (68) from its retracted to its extended position is effective to (i) move the staple holder with respect to the staple housing toward the anvil (A1), (ii) move the anvil member toward the staple member (A2), and (iii) move the anvil with respect to the anvil housing toward the staple holder (A3).
3. The device of paragraph 2, wherein the anvil member (27) includes drive links (114) operatively connected to the arm assembly (32) for moving the anvil (96) toward the staple holder (78) (A3) as the drive assembly (106) is moved from its retracted to its extended position.
4. The device of paragraph 1, wherein the staple holder (78) and anvil (96) have confronting surfaces that define, in combination with arm assembly (32), a chamber (21), and the effect of movement of the drive member (68) from its retracted to its extended position is to squeeze tissue captured within the chamber to form a captured tissue fold.
5. The device of paragraph 4, wherein the chamber (21) is covered by a membrane (24), allowing tissue to be drawn into the chamber, with application of a vacuum to the membrane.
6. The device of paragraph 5, wherein the membrane (24) has an opening (26) on one .side thereof, for sucking tissue into the chamber, and movement of the drive member (68) from its retracted to extended positions, with application of vacuum to the chamber, is effective to draw tissue into the side of the chamber opposite the opening.
7. The device of paragraph 4, wherein the drive member includes a disc (68) that travels within the staple housing (25), and carries at least one pin (84) that moves within a slot (64) in the staple housing (25), limiting the extent of travel of the drive member toward its extended position to the extent of travel allowed for the pin (84) within the slot (64).
8. The device of paragraph 7, which further includes at least one arm spreader (113) pivotally connecting said disc to the arm assembly (32), for spreading the arm assembly outwardly as the disc travels from its retracted to its extended position.
9. The device of paragraph 1, wherein the drive member includes a drive piston (106) connected to a disc (68), and which further includes a staple piston (1 16) carried for movement within the drive piston (106) between retracted and extended positions and a staple pusher (76) adapted to engage one or more staples in the staple holder (78) and eject the one or more staples from the holder against the anvil (96), when the staple pusher is moved with the staple piston from a retracted to extended position.
10. The device of paragraph 9, for use with a staple holder (78) designed to hold an annular array of staples, and the staple pusher (78) is designed to engage and eject the array of staples in the holder simultaneously.
11. The device of paragraph 10, wherein the staple holder (78) is a replaceable staple cartridge adapted to be inserted into the staple housing to travel therein with the drive member (68) between retracted and extended positions.
12. The device of paragraph 11, wherein the drive member includes a disc (68) having at least one axially extending post (84) adapted to engage the staple cartridge, with such received in the device, and prevent angular movement of the cartridge within the staple housing.
13. The device of paragraph 12, which further includes a cartridge-side reinforcing ring (83) disposed against the cartridge (78), and having openings (85) for receiving staples therethrough, for attaching the reinforcing ring (83) to the side of stapled tissue facing the cartridge.
14. The device of paragraph 9, which further includes a tissue cutter (86) mounted on the staple piston (116), adapted to cut a hole in a tissue fold held between the staple holder (78) and anvil (96), as the tissue fold is being stapled by movement of the staple piston (116) from its retracted to its extended position.
15. The device of paragraph 13, wherein the anvil member includes a compressible cutting board (99a, 99d), allowing the cutter (86) to be advanced by movement of the staple piston (116) beyond its point of initial contact with the cutting board.
16. The device of paragraph 15, wherein the cutting board (99a) is formed of a material, such as silicone, that can be penetrated by the cutter.
17. The device of paragraph 15, wherein the cutting board (99d) is spring biased in the direction opposing movement of the cutter.
18. The device of paragraph 9, which further includes an alignment pin (160, 168) carried on one of the staple and anvil members, and a pin-receiving bushing (164, 170) carried on the other of the members, said pin and bushing being positioned such that movement of the staple piston toward its extended position causes the pin to mate with the bushing, thus to hold the two members in axial alignment as staples are ejected with further movement of the staple piston (116) toward its extended position.
19. The device of paragraph 18, wherein one of the pin and pin-receiving bushing is retractable under a spring bias.
20. The device of paragraph 1 which is carried at the distal end of a shaft (16) carrying hydraulic fluid to the device, wherein the staple and anvil members of the device are proximal and distal members, respectively, with the shaft being operatively connected to the proximal member of the device.
21. A medical instrument (10) for stapling stomach tissue comprising
   the stapling device (14) of paragraph 1,
   a shaft (16) having a proximal-end handle and a distal end,
   an articulating section (128) connecting the distal end of the shaft to the first, proximal member of the stapling device, and
   a hydraulic fluid line (130) contained within the shaft effective for carrying hydraulic fluid under a pressure of at least about 1,000 psi to the drive mechanism in the staple member, wherein the hydraulic fluid line within the articulating section has a coiled or sinusoidal configuration, to accommodate off-axis movement of the device with respect to the shaft.
22. The instrument of paragraph 21, wherein the articulating section (128) has a spine (128) formed of a plurality of links (132) formed over the coiled or sinusoidal portion of then hydraulic line (130).
23. The instrument of paragraph 21 , wherein the stapling device includes a hydraulically driven drive member (68) and a hydraulically driven staple piston ( 1 16), said shaft ( 16) and articulating section ( 128) carry separate hydraulic lines ( 130) for the drive member and staple piston, and the lines ( 130) have an interleave coil configuration within the articulating section.
24. A tissue capture device (14) for capturing and immobilizing a tissue fold, comprising
   a first member (25) having a housing (30) and a first tissue-contact plate (78) independently movable within the first-member housing,
   a second member (27) having a housing (30) and a second tissue-contact plate (96) independently movable within the second-member housing,
   a drive assembly (29) including a drive member (68) operatively connected to the first tissue-contact plate (78) for movement within the first-member housing (28) from a retracted position to an extended position, and an arm assembly (32) operatively coupled to the first and second members (25,27), such that movement of the drive member (68) from its retracted to its extended position is effective to (i) move the tissue-contact plate (78) within the first-member housing toward the second-tissue contact member (96) (A1), and (ii) move the anvil member (27) toward the staple member (A2),
   an elastomeric membrane extending between the two members and defining a tissue capture chamber between the two tissue-contact plates, and having an opening therein for drawing tissue into the chamber, upon application of a vacuum to the chamber, and
   an expansion member (37) operatively coupled to the two members for expanding the chamber membrane outwardly, on the side of the membrane opposite said opening, upon movement of the drive member from its retracted to its expanded condition, causing additional tissue to be drawn into the chamber between the two tissue-contact plates, until the tissue is clamped between two tissue-contact plates.
25. A method for capturing and stapling a tissue fold, by the steps of:
   (a) drawing a tissue fold into a vacuum chamber (21) defined by first and second relatively movable members (25, 27) and an elastomeric membrane (24) extending between the two,
   (b) advancing a staple holder (78) and anvil (96) contained within the first and second members (25, 27), respectively, toward one another by independent movement of the staple holder and anvil within its associated member, respectively, and by moving the second housing (27) toward the first housing (25);
   (c) by continued application of vacuum during said advancing, continuing to draw tissue into said chamber (21) until a tissue fold is captured between the staple holder and anvil, and
   (d) stapling the captured tissue fold.
26. A medical instrument (10) for stapling stomach tissue comprising
   the stapling device (14) of paragraph 1,
   a shaft (16) having a proximal-end handle and a distal end,
   an articulating section (128) connecting the distal end of the shaft to the first, proximal member of the stapling device, and
   a hydraulic fluid line (130) contained within the shaft capable of carrying hydraulic fluid under a pressure of up to 1,500 psi or greater, to the drive mechanism (106) in the staple member (25), wherein the hydraulic fluid line within the articulating section has a coiled or sinusoidal configuration, to accommodate off-axis movement of the device with respect to the shaft.
27. The instrument of paragraph 26, wherein the articulating section (128) has a spine (128) formed of a plurality of links (132) formed over the coiled or sinusoidal portion of the hydraulic line (130).
28. The instrument of paragraph 26, wherein the stapling device includes a hydraulically driven drive member (68) and a hydraulically driven staple piston (116), and the shaft (16) and articulating section (128) carry separate hydraulic lines (130) for the drive member and staple piston, and the lines (130) have an interleaved coil configuration within the articulating section.
29. In a medical instrument (12) having a hydraulically driven device (14) for capturing and/or stapling tissue and an elongate shaft (16) for delivering hydraulic fluid to the device, a snap-on connection between the distal end of a shaft and the proximal face of a staple member in a hydraulically activated staple device, comprising
   at the distal shaft end, a plate having one or more openings for supplying hydraulic fluid under pressure from hydraulic lines attached to the openings at the input side of the plate, and an engagement edge adjacent each opening,
   a plate-receiving face on the staple device including correspondingly positioned openings for receiving fluid into the device, where the openings communicate at the output side of the face with hydraulic supply lines within the staple device, and an undercut boss adjacent each opening, such that when the shaft plate is placed against the staple-device face, and rotated slightly to wedge the engagement edge(s) on the shaft plate within the undercut regions of the bosses of the device plate, an O-ring located between the plate and face at each opening is compressed, thus sealing the connection between the aligned openings, and
   a lock mechanism on the shaft plate that engages the device face to lock the plate against rotational movement once the fluid-supply opening are received.
30. The stapler device (14) for applying a staple to a tissue, comprising
   a first, staple member (25) having a staple housing (28) and a staple holder (78),
   a second, anvil member (27) having an anvil housing (30) and an anvil (96), and
   a drive assembly including (i) a drive member (68) for movement within the staple housing (28) from a retracted position to an extended position, (ii) attached to the drive member, one or more pins (84) that travel within one or more slots (64) formed in the staple housing, limiting movement of the drive member between retracted arid extended positions to the limits of travel of said pin(s) in said slot(s), (iii) an arm assembly (32) operatively coupled to the staple and anvil members, such that movement of (lie drive member (68) from its retracted toward its extended position is effective to move the staple holder toward the anvil (A1), and (iv) at least one arm spreader (113) pivotally connecting said drive member to the arm assembly, for spreading the arm assembly outwardly as the drive member travels from its first to its second position.
31. The device of paragraph 30, wherein the arm spreader (113) is pivotally connected to the pin and the arm assembly, such that movement of the pin within its slot in a retracted to extended position is effective to move the arm assembly outwardly, wherein the arm spreader provides a truss-like support between the staple body and the associated assembly arm.
32. The device of paragraph 31, wherein drive member (68) is effective to advance the staple holder (78) within its housing (28), as the drive member is moved from its retracted to extended positions, and the arm assembly (32) may be effective to advance the anvil (96) within its housing as (he drive member is moved from its retracted to extended positions.
33. A device (14) for forming a cut within a tissue, comprising
   a first member 25 having a housing 28 and drive piston (1 16) and a cutter (86) attached to the piston for movement therewith within between retracted and extended positions, and
   a second member having a housing (30) and a cutting board (99), where the cutter (86) contacts the cutting board (99) to form a hole in a tissue disposed between the two members, and the cutting board (99) has a resilient cutting surface that allows the cutter (86) to advance in the direction of the cutting board (99) beyond the initial point of contact therewith.
34. The device of paragraph 33, wherein the cutting board (99a) is formed of a material, such as silicone, that can be penetrated by the cutter.
35. The device of paragraph 33, wherein the cutting board (99d) is spring biased in the direction opposing movement of the cutter toward its extended position.
36. A stapler device (14) for applying a staple to a tissue, comprising
   a staple member having a first or staple housing (28) and a staple holder (78),
   a second, anvil member having a second, anvil housing (30) and an anvil (96),
   a drive assembly including (i) a drive member (68) for movement within the staple member housing from a retracted position to an extended position, to move the staple holder adjacent the anvil for capturing tissue between the holder and anvil, a staple piston (116) in the first member, movable between first, retracted and second, extended positions in the first member for driving staples from the holder through the tissue against the anvil,
   an alignment pin (160,168) carried on one of the staple and anvil members, and
   a pin-receiving bushing (164, 170) carried on the other of the members, where the pin and bushing are positioned such that movement of the staple piston toward its extended position causes the pin to mate with the bushing, thus to hold the two members in axial alignment as staples are ejected with further movement of the staple piston (116) toward its extended position.
37. The device of paragraph 36, wherein one of the pin (160,168) and pin-receiving bushing (164, 170) is retractable under a spring bias.
38. A tissue capture device (14) for capturing and immobilizing a tissue fold, comprising
   a first member (25) having a housing (30) and a first tissue-contact plate (78) independently movable within the first-member housing,
   a second member (27) having a housing (30) and a second tissue-contact plate (96) independently movable within the second-member housing,
   a drive assembly (29) in the device includes a drive member (68) operatively connected to the first tissue-contact plate (78) for movement within the first-member housing (28) from a retracted position to an extended position, and an arm assembly (32) operatively coupled to the first and second members (25, 27), such that movement of the drive member (68) from its retracted to its extended position is effective to (i) move the tissue-contact plate (78) within the first-member housing toward the second-tissue contact member (96) (A1), and (ii) move the anvil member (27) toward the staple member (A2),
   a membrane extends between the two members, defining a tissue capture chamber between the two tissue-contact plates, and having an opening therein for drawing tissue into the chamber, upon application of a vacuum to the chamber, and
   an expansion member or raiser (37) operatively coupled to the two members operates to expand for the chamber membrane outwardly, on the side of the membrane opposite the opening, upon movement of the drive member from its retracted to its expanded condition, causing additional tissue to be drawn into the chamber between the two tissue-contact plates, until the tissue is captured by clamping between two tissue-contact plates.
39. A method for capturing a tissue fold by the steps of:
   (a) drawing tissue (17) into a vacuum chamber (21) defined by first and second relatively movable members (25, 27) and an elastomeric membrane 24) extending between the two,
   (b) advancing a first tissue-contact plate (78) and a second tissue -contact plate (96) contained within the first and second members, respectively, toward one another by independent movement of the first and second tissue -contact plates (78, 96) staple within their associated members (25, 27), respectively, and by moving the second member toward the first member; and
   (c) by continued application of vacuum during said advancing, continuing to draw tissue into said chamber untie a tissue fold (1 7a) is captured between the first and second tissue-contact plates.

## Claims

1. A surgical stapler device (14) for applying a staple to a tissue, comprising,
a staple member (25) having a staple housing (28) and a staple holder (78);
an anvil member (27) having an anvil housing (30) and an anvil (96) carried on the anvil housing (30),
a drive assembly (29) including a drive member (68) operatively connected to the staple holder (78) for movement within the staple housing (28) from a retracted position to an extended position, and
an arm assembly (32) operatively coupled to the staple and anvil members (25, 27), **characterised by**
at least a first and a second spreader arm (113) coupled to the drive assembly (29) and the arm assembly (32) such that movement of the drive member (68) from its retracted to its extended position is effective to move the spreader arms (113) outwardly and thus to move the arm assembly (32) outwardly.

2. The device of claim 1, the drive member (68) further comprising at least one pin (84) operatively connected to the drive member (68) and one of the arm spreaders (113) that travels within a slot (64) in the staple member (25) from a retracted to an extended position, wherein movement of the at least one pin from its retracted to extended position causes the arm spreaders (113) to pivot relative to the pin (84) and drive the associated arm assembly (32) outwardly.

3. The device of claim 1, wherein the staple holder (78) is independently movable with respect to the staple housing (28) such that movement of the drive member (68) from its retracted to extended position is effective to move the staple holder (78) with respect to the staple housing (28).

4. The device of claim 3, wherein the anvil (96) is independently movable in the anvil housing (30), and the arm assembly (32) is operatively coupled to the anvil (96), such that movement of the drive member (68) from its retracted to its extended position is effective to (i) move the staple holder (78) with respect to the staple housing (28) toward the anvil (96), (ii) move the anvil member (27) toward the staple member (25), and (iii) move the anvil (96) with respect to the anvil housing (30) toward the staple holder (78).

5. The device of claim 4, wherein the anvil member (27) includes drive links (114) operatively connected to the arm assembly (32) for moving the anvil (96) toward the staple holder (78) as the drive assembly (29) is moved from its retracted to its extended position.

6. The device or claim 1, wherein the staple holder (78) and anvil (96) have confronting surfaces that define, in combination with arm assembly (32), a chamber (21), and the effect of movement of the drive member (68) from its retracted to its extended position is to squeeze tissue captured within the chamber to form a captured tissue fold.

7. The device of claim 6, wherein the chamber (21) is covered by a membrane (24), allowing tissue to be drawn into the chamber, with application of a vacuum to the membrane.

8. The device of claim 7, wherein the membrane (24) has an opening (26) on one side thereof, for drawing tissue into the chamber, and movement of the drive member (68) from its retracted to extended positions, with application of vacuum to the chamber, is effective to draw tissue into the side of the chamber opposite the opening.

9. The device of claim 1, wherein the drive member includes a drive piston (106) connected to the drive member (68), and which further includes a staple piston (116) carried for movement within the drive piston (106) between retracted and extended positions and a staple pusher (76) adapted to engage one or more staples in the staple holder (78) and eject the one or more staples from the holder against the anvil (96), when the staple pusher is moved with the staple piston from a retracted to extended position.

10. The device of claim 9, for use with the staple holder (78) designed to hold an annular array of staples, and the staple pusher (76) is designed to engage and eject the array of staples in the holder simultaneously.

11. The device of claim 10, wherein the staple holder (78) is a replaceable staple cartridge adapted to be inserted into the staple housing to travel therein with the drive member (68) between retracted and extended positions.

12. The device of claim 1, which further includes a reinforcing ring (83) disposed against the staple holder (78), and having openings (85) for receiving staples therethrough, for attaching the reinforcing ring (83) to the side of stapled tissue facing the staple holder.

13. The device of claim 9, which further includes a tissue cutter (86) mounted on the staple piston (116), adapted to cut a hole in a tissue fold held between the staple holder (78) and anvil (96), as the tissue fold is being stapled by movement of the staple piston (116) from its retracted to its extended position.

14. The device of claim 13, wherein the anvil member includes a compressible cutting board (99a, 99d), allowing the cutter (86) to be advanced by movement of the staple piston (116) beyond its point of initial contact with the cutting board.

15. The device of claim 9, which further includes an alignment pin (160, 168) carried on one of the staple and anvil members, and a pin-receiving bushing (164, 170) carried on the other of the members, said pin and bushing being positioned such that movement of the staple piston toward its extended position causes the pin to mate with the bushing, thus to hold the two members in axial alignment as staples are ejected with further movement of the staple piston (116) toward its extended position.

16. A medical instrument (10) for stapling stomach tissue comprising
the stapler device (14) of any one of claims 1-15,
a shaft (16) having a proximal-end handle and a distal end,
an articulating section (128) connecting the distal end of the shaft to the staple member of the stapler device, and
a hydraulic fluid line (130) contained within the shaft effective for carrying hydraulic fluid under a pressure of at least about 6900 kPa (about 1,000 psi) to a drive mechanism in the staple member, wherein the hydraulic fluid line within the articulating section has a coiled or sinusoidal configuration, to accommodate off-axis movement of the device with respect to the shaft.

17. The instrument of claim 16, wherein the articulating section (128) has a spine formed of a plurality of links (132) formed over the coiled or sinusoidal portion of the hydraulic line (130).

18. The instrument of claim 16, wherein the drive member (68) is hydraulically driven and the stapler device includes a hydraulically driven staple piston (116), said shaft (16) and articulating section (128) carry separate hydraulic lines (130) for the drive member and staple piston, and the lines (130) have an interleaved coil configuration within the articulating section.
